(19) Europäisches Patentamt
European Patent Office
Office européen des brevets

(11) Publication number: **0 095 778**
**B1**

(12) **EUROPEAN PATENT SPECIFICATION**

(45) Date of publication of patent specification: **30.07.86**

(21) Application number: **83105395.4**

(22) Date of filing: **31.05.83**

(51) Int. Cl.[4]: **C 07 D 417/12,** A 61 K 31/425 // (C07D417/12, 205:08, 277:40)

(54) **2-Oxo-1-azetidinesulfonic acid derivatives, process for production thereof, and use thereof.**

(30) Priority: **31.05.82 JP 91471/82**
**24.03.83 JP 47852/83**

(43) Date of publication of application:
**07.12.83 Bulletin 83/49**

(45) Publication of the grant of the patent:
**30.07.86 Bulletin 86/31**

(84) Designated Contracting States:
**AT BE CH DE FR GB IT LI LU NL SE**

(56) References cited:
**EP-A-0 021 678**
**EP-A-0 048 953**
**EP-B-0 002 482**

**CHEMICAL ABSTRACTS, vol. 96, no. 21, May 24, 1982, Columbus, Ohio, USA, SQUIBB, E.R. and Sons, Inc., "Antibiotic beta-lactams", page 690, column 2, abstract no. 181062x**

**The file contains technical information submitted after the application was filed and not included in this specification**

(73) Proprietor: **BANYU PHARMACEUTICAL CO., LTD.**
**7-8, Nihonbashi Honcho 2-chome**
**Chuo-ku Tokyo 103 (JP)**

(72) Inventor: **Nakagawa, Susumu**
**10-1, Shingu-cho**
**Okazaki-shi Aichi-ken (JP)**
Inventor: **Nakano, Fumio**
**4-63, Rokku-cho**
**Okazaki-shi Aichi-ken (JP)**
Inventor: **Ushijima, Ryosuke**
**14-3, Tosakimoto-machi**
**Okazaki-shi Aichi-ken (JP)**
Inventor: **Iwatsuki, Ikuo**
**102, Sakae Take-machi**
**Toyota-shi Aichi-ken (JP)**
Inventor: **Iwadare, Shuichi**
**301, Itopia Higashihiroo 3-19 Minamiazabu**
**Minato-ku Tokyo (JP)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel azetidinesulfonic acid derivatives and their salts. This invention also relates to a process for producing these compounds, and their use as antibacterial agents.

More specifically, this invention relates to an optically active compound represented by the following formula (1)

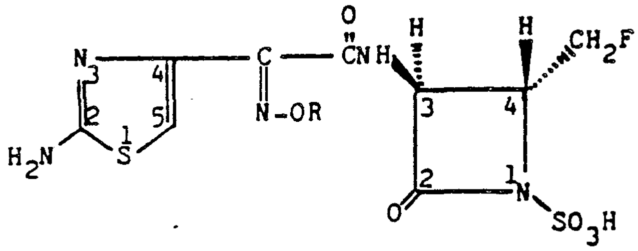

...(1)

wherein R represents a group selected from the class consisting of linear or branched $C_1$—$C_4$ alkyl groups, $C_3$—$C_4$ alkenyl groups, $C_3$—$C_4$ alkynyl groups, a cyanomethyl group, $C_3$—$C_6$ cycloalkyl groups (the latter being optionally substituted by carboxy), carboxy-substituted $C_1$—$C_5$ alkyl groups, a benzyl group and a phenyl group;

and a pharmaceutically acceptable salt thereof. The invention also relates to an antibacterial composition comprising the compound of formula (1) and a pharmaceutically acceptable salt thereof and a process for producing the compound of formula (1) and its pharmaceutically acceptable salt.

As shown by formula (1), the novel compounds of this invention have such a stereochemical structure that an α-fluoromethyl group is joined at the 4-position and a β-acylamino group at the 3-position of the 2-azetidinone ring in a trans configuration (3S, 4R). The oxyimino in the acylamino moiety at the 3-position of the 2-azetidinone ring generally can take an E or Z geometric structure, but the compounds of this invention has a Z structure as shown in formula (1). The novel compounds of this invention have =N—OR defined above in formula (1) having the aforesaid structural characteristics. The compounds of formula (1) and their pharmaceutically acceptable salt in accordance with this invention are novel.

Recently, monocyclic β-lactam antibacterial substances having a 2-azetidinone skeleton with an acylamino group at the 3-position and a sulfonic acid group at the 1-position were discovered independently by A. Imada et al. [Nature, *289*, 590 (1981)] and R. B. Sykes et al. [Nature, *291*, 489 (1981)], and were named monobactam by the latter. A number of monobactam derivatives have been produced from natural β-lactams or by synthetic means. For example, before the first priority date of the present application, such compounds were proposed in Japanese Laid-Open Patent Publications Nos. 164671/1980 (laid-open on December 22, 1980), 164672/1980 (laid-open on December 22, 1980), 125362/1981 (laid-open on October 1, 1981; corresponding to G. B. Patent No. 2071650), 133259/1981 (laid-open on October 18, 1981), 133260/1981 (October 19, 1981), 135465/1981 (October 22, 1981), 138169/1981 (laid-open on October 28, 1981) and 139454/1981 (laid-open on October 30, 1981).

Furthermore, after the first priority date but before the second priority date of the present application, such compounds were proposed in Japanese Laid-Open Patent Publications Nos. 98258/1982 (laid-open on June 18, 1982), 118560/1982 (laid-open on July 23, 1982), 131758/1982 (laid-open on August 14, 1982), 131759/1982 (laid-open on August 14, 1982), 175162/1982 (laid-open on October 28, 1982; corresponding to European Patent No. 61763), 212179/1982 (laid-open on December 27, 1982), 8060/1983 (laid-open on January 18, 1983), and 18381/1983 (laid-open on February 2, 1983).

However, these patent documents quite fail to describe the compounds of formula (1) and their pharmaceutical acceptable salts in accordance with this invention.

The present inventors discovered that the novel compounds of formula (1) and their pharmaceutically acceptable salts can exist, and succeeded in synthesizing these compounds. The present inventors also found that these compounds have excellent antibacterial activity, and are very useful as antibacterial agents.

Investigations of the present inventors have shown that the compounds of formula (1) and their pharmaceutically acceptable salts can be produced easily in good yields, and these compounds are useful for the treatment and prevention of bacterial infections.

It is an object of this invention therefore to provide novel compounds of formula (1) and their pharmaceutically acceptable salts.

A second object of this invention is to provide a process for producing the aforesaid compounds.

A third object of this invention is to provide an antibacterial agent comprising such a compound as an active ingredient.

The above and other objects and advantages of this invention will become more apparent from the following description.

The optically active compounds of this invention are represented by the following formula (1).

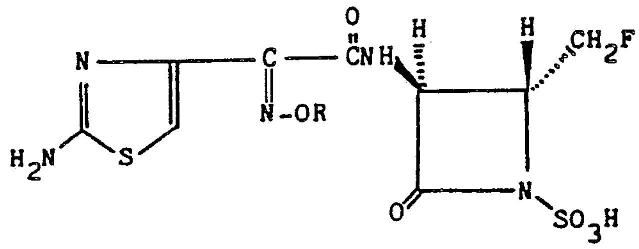

...(1)

wherein R is a group selected from the class consisting of linear or branched $C_1$—$C_4$ alkyl groups, $C_3$—$C_4$ alkenyl groups, $C_3$—$C_4$ alkynyl groups, a cyanomethyl group, $C_3$—$C_6$ cycloalkyl groups (the latter being optionally substituted by carboxy), carboxy-substituted $C_1$—$C_5$ alkyl groups, a benzyl group and a phenyl group.

The compounds of formula (1) can be in the form of their pharmaceutically acceptable salts.

Examples of the $C_1$—$C_4$ alkyl groups for R in formula (1) are methyl, ethyl, n-propyl, isopropyl, n-butyl, and isobutyl. Examples of the $C_3$—$C_4$ alkenyl or alkynyl groups for R in formula (1) are 2-propenyl, 2-butenyl, 2-propynyl and 2-butynyl. Examples of $C_3$—$C_6$ cycloalkyl groups which may be substituted by carboxy include cyclopropyl, cyclobutyl, cyclopentyl, cyclohexyl, 1-carboxy-1-cyclopropyl, 1-carboxy-1-cyclobutyl, 1-carboxy-1-cyclopentyl and 1-carboxy-1-cyclohexyl. Examples of the carboxy-substituted $C_1$—$C_5$ lower alkyl groups for R in formula (1) are carboxymethyl, 1-carboxy-1-methylethyl, 1-carboxy-1-methylpropyl, 1-carboxy-1-methylbutyl, and 1-carboxy-1-ethylpropyl.

Preferred examples of the group R are linear or branched $C_1$—$C_3$ alkyl groups, a $C_3$ alkenyl or alkynyl group, carboxy- or cyano-methyl, cyclopentyl, 1-carboxy-1-($C_3$—$C_6$ cycloalkyl groups), and 1-carboxy-1-methyl ($C_2$—$C_3$ alkyl groups).

Specific examples of the optically active compounds of formula (1) in accordance with this invention are given below.

(3S, 4R) - (−) - 3 - [(Z) - 2 - (2 - amino - 4 - thiazolyl) - 2 - methoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - ethoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - n - propoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - isopropoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - n - butoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - isobutoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (2 - propenyloxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (2 - butenyloxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (2 - propynyloxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (2 - butynyloxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - carboxymethoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - cyanomethoxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - cyclopropyloxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - cyclobutyloxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - cyclopentyloxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - cyclohexyloxyiminoacetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxy - 1 - cyclopropoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxy - 1 - cyclobutoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxy - 1 - cyclopentoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxyl - 1 - cyclohexyloxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxy - 1 - methylethoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxy - 1 - methylpropoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - (1 - carboxyl - 1 - methylbutoxyimino)acetamide] - 4 - fluoromethyl - 2 - oxo - 1 - azetidinesulfonic acid,

(3S, 4R) - (−) - 3 - [(Z) - 2 - (2 - amino - 4 - thiazolyl) - 2 - benzyloxyiminoacetamide] - 4 - fluoromethyl - 2 - oxo - 1 - azetidinesulfonic acid, and

(3S, 4R) - (−) - 3 - [(Z) - 2 - (2 - amino - 4 - thiazolyl) - 2 - phenoxyiminoacetamide] - 4 - fluoromethyl - 2 - oxo - 1 - azetidinesulfonic acid.

The pharmaceutically acceptable salts of these exemplified compounds can also be cited. Examples of such salts are inorganic and organic acid salts. Specific examples of the salts are alkali metal salts such as sodium and potassium salts, alkaline earth metal salts such as calcium and magnesium salts, inorganic amine salts such as an ammonium salt, organic amine salts such as trimethylamine, triethylamine, N,N′-dibenzylethylenediamine tetrabutylammonium, and procaine salts, inorganic acid salts such as hydrochloric acid, hydrobromic acid, sulfuric acid, phosphoric acid and nitric acid salts, organic acid salts such as acetic acid, lactic acid, propionic acid, maleic acid, malic acid, tartaric acid, citric acid, methanesulfonic acid and isethionic acid salts, and amino acid salts such as arginine, lysine, aspartic acid and glutamic acid salts.

The optically active compound of formula (1) and its pharmaceutically acceptable salt can be produced by a process which comprises reacting a compound represented by the following formula (A)

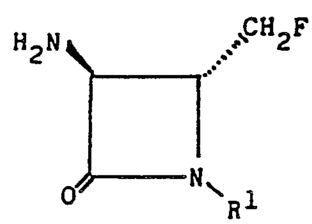

...(A)

wherein $R^1$ represents a hydrogen atom, $-SO_3H$ or an inorganic salt or organic amine salt of $-SO_3H$, with a reactive derivative resulting from the activation of the carboxyl group ($-COOH$) of a compound represented by the following formula (B)

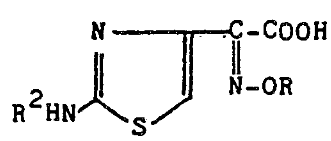

...(B)

wherein $R^2$ represents a hydrogen atom or a protective group for the amino group, and R is the same as defined with regard to formula (1) provided that when R has a carboxy substituent in said definition, it is protected by a protective group for the carboxy;
when $R^1$ in the compound of formula (A) is a hydrogen atom, sulfonating the reaction product; and then eliminating the protective group.

When $R^1$ in the compound (A) is $-SO_3H$, examples of its inorganic salt or organic amine salt include inorganic alkali salts, for example such alkali metal salts as lithium, sodium and potassium salts and such alkaline earth metal salts as calcium and magnesium salts; and organic amine salts, for example trialkylamine salts such as trimethylamine and triethylamine salts, and quaternary ammonium salts such as pyridine, picoline, lutidine and tetra-n-butyl ammonium salts.

The protective group for the amino group, which is represented by $R^2$ in formula (B), may be a hydrogen atom or an ordinary N-protective group. Examples include substituted or unsubstituted ar(lower)alkyl groups such as benzyl, benzhydryl, 4-methoxybenzyl and 3,4-dimethoxybenzyl, a tetrahydropyranyl group, substituted phenylthio groups, substituted alkylidene groups, substituted aralkylidene groups, substituted cycloalkylidene groups and acyl groups. Specific examples of acyl groups suitable as N-protective groups are substituted or unsubstituted lower alkanoyl groups such as formyl, acetyl, chloroacetyl and trifluoroacetyl; substituted or unsubstituted lower alkoxycarbonyl groups such as methoxycarbonyl, ethoxycarbonyl, propoxycarbonyl, 1-cyclopropylethoxycarbonyl, isopropoxycarbonyl, butoxycarbonyl, t-butoxycarbonyl, pentyloxycarbonyl, t-pentyloxycarbonyl, hexyloxycarbonyl, trichloroethoxycarbonyl, and 2-pyridylmethoxycarbonyl; substituted or unsubstituted ar(lower)alkoxycarbonyl groups such as benzyloxycarbonyl, benzhydryloxycarbonyl, and 4-nitrobenzyloxycarbonyl; an 8-quinolyloxycarbonyl group; a succinyl group; and a phthaloyl group. The reaction product between an amino group and a silicon or phosphorus compound is also within the scope of N-protective groups. Such a silicon or phosphorus compound is, for example, trimethylsilyl chloride or diphenylphosphinic chloride.

When R in the compound of formula (B) has a carboxy substituent, the protective group for the carboxy is, for example, an ester group.

Examples of such ester groups include alkyl esters such as methyl, ethyl, propyl, butyl and t-butyl esters; alkenyl esters such as vinyl and allyl esters; alkynyl esters such as ethynyl and propynyl esters; alkoxyalkyl esters such as methoxymethyl, ethoxymethyl, isopropoxymethyl, 1-methoxyethyl and 1-ethoxyethyl esters; alkylthioalkyl esters such as methylthiomethyl, ethylthiomethyl, isopropylthiomethyl and ethylthioethyl esters; haloalkyl esters such as 2-iodoethyl and 2,2,2-trichloroethyl esters; alkanoyloxyalkyl esters such as acetoxymethyl, propionyloxymethyl, pivaloyloxymethyl, 2-acetoxyethyl and 2-propionyloxymethyl esters; alkanesulfonyl-alkyl esters such as mesylmethyl and 2-mesylethyl esters; and substituted or unsubstituted aralkyl esters such as benzyl, 4-methoxybenzyl, 4-nitrobenzyl, phenethyl, trityl, benzhydryl, bis(methoxyphenyl)methyl and 3,4-dimethoxybenzyl esters.

Examples of the reactive derivatives resulting from the activation of the carboxyl group (—COOH) of the compound of formula (B) are acid chlorides, active esters, active amides, and acid anhydrides. Methods for forming such reactive derivatives are known *per se*. The acid chlorides can be obtained by contacting the compound of formula (B) with a suitable chlorinating agent such as phosphorus pentachloride, phosphorus trichloride, thionyl chloride, oxalyl chloride, phosgene and Vilsmeier reagent.

Examples of the active esters are methyl, ethyl, methoxymethyl, propargyl, 4-nitrophenyl, 2,4-dinitrophenyl, trichlorophenyl, pentachlorophenyl and mesylphenyl esters; and esters with 1-hydroxy-2-(H)-pyridone, N-hydroxysuccinimide, N-hydroxyphthalimide, 1-hydroxybenzotriazole, or N-hydroxy-5-norbornene-2,3-dicarboximide. These esters can be obtained, for example, by treating the compound of formula (B) with alcohols, phenols, or N—OH compounds together with condensing agents such as DCC or acid catalysts such as hydrochloric acid, sulfuric acid and p-toluenesulfonic acid.

The active amide may be acid amides such as pyrazole, imidazole, 4-substituted imidazole, dimethylpyrazole, triazole, tetrazole, and benzotriazole. They can be obtained, for example, by treating the compound of formula (B) with reagents such as carbonyldiimidazole.

Examples of the acid anhydrides are substituted phosphoric acid mixed anhydrides (e.g., dialkylphosphoric acid mixed anhydrides, phenylphosphoric acid mixed anhydrides, diphenylphosphoric acid mixed anhydrides, dibenzylphosphoric acid mixed anhydrides and halogenated phosphoric acid mixed anhydrides), dialkylphosphorous acid mixed anhydrides, sulfurous acid mixed anhydrides, thiosulfuric acid mixed anhydrides, sulfuric acid mixed anhydrides, alkylcarbonic acid mixed anhydrides, aliphatic carboxylic acids (e.g., pivalic acid, pentanoic acid, isopentanoic acid, 2-ethylbutanoic acid, or trichloroacetic acid) mixed anhydrides, aromatic carboxylic acid (e.g., benzoic acid) mixed anhydrides, and symmetrical acid anhydrides. These acid anhydrides can be obtained, for example, by treating an inorganic salt or an organic amine salt of the compound of formula (B) with a reactive derivative of the corresponding compound.

The acid chloride can be formed, for example, by reacting the compound of formula (B) with 1 to 10 times, preferably 1 to 1.5 times, its amount in moles of a chlorinating agent at a reaction temperature or −40 to 100°C, preferably −20 to 20°C, in a solvent or a solvent mixture which does not adversely affect the reaction (e.g., methylene chloride, chloroform, dichloroethane, benzene and toluene).

The active esters can be formed by reacting the compound of formula (B) with 1 to 1.2 times its amount in moles of the corresponding alcohol, phenol or N—OH compound at a reaction temperature of −30 to 40°C, preferably −10 to 25°C. in the presence of 1 to 1.2 moles, per mole of the compound of formula (B), of a condensing agent in a solvent or solvent mixture which does not adversely affect this reaction. Examples of the solvent are acetone, dioxane, acetonitrile, tetrahydrofuran, methylene chloride, chloroform, ethyl acetate, and N,N-dimethylformamide. Examples of the condensing agent include N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisopropylcarbodiimide and N-ethyl-N'-(3-dimethylaminopropyl)carbodiimide.

The active amide can be formed by reacting the compound of formula (B) with 1 to 1.2 times its amount in moles of a reactive derivative such as carbonyldiimidazole at a reaction temperature of −30 to 40°C, preferably 0 to 25°C, in a solvent or a solvent mixture which does not adversely affect the reaction (e.g., acetone, dioxane, acetonitrile, methylene chloride, chloroform, benzene, ethyl acetate, or N,N-dimethylformamide.

The acid anhydride can be formed by reacting an inorganic salt or organic amine salt of the compound of formula (B) with 1 to 1.1 times its amount in moles of diphenylphosphoryl chloride, sulfuric anhydride, alkyl chloroformates, aliphatic carboxylic acid chlorides, etc. in a solvent or solvent mixture which does not adversely affect the reaction (e.g., acetone, dioxane, acetonitrile, tetrahydrofuran, methylene chloride, chloroform, benzene, ethyl acetate, and N,N-dimethylformamide) at a reaction temperature of −30 to 20°C, preferably −15 to 0°C.

The compound of formula (1) in accordance with this invention can be easily produced by reacting the compound of formula (A) with the reactive derivative of the compound of formula (B) resulting from activation of the carboxyl group, —COOH, of the latter, and when $R^1$ in the compound of formula (A) is a

hydrogen atom, sulfonating the reaction product, and then eliminating the protective group $R^2$. When R in the compound of formula (B) is a protective carboxyl group, the protective group for the carboxyl group is eliminated simultaneously with the elimination of $R^2$. It is not necessary therefore to eliminate the protective groups in a multiplicity of steps.

The reaction of the compound of formula (A) with the reactive derivative of the compound of formula (B) can be carried out as follows:

Reaction conditions such as reagents and solvents used in the reaction and the reaction temperature can be properly selected depending upon the types of the compound of formula (A) and the reactive derivative of the compound of formula (B). For example, when the active ester or active amide is used as the reactive derivative, it is used in an amount of 1 to 1.5 moles per mole of the compound of formula (A) with or without isolating it from the reaction mixture in the preparation of the reactive derivative. The reaction may be carried out at −30 to 40°C, preferably −10 to 30°C. Under these conditions, the reaction proceeds easily.

When the reactive derivative of the compound of formula (B) is the acid chloride or acid anhydride, the reaction is desirably carried out in the presence of an alkali metal carbonate, or an organic amine, for example a trialkylamine such as trimethylamine or triethylamine, and N-methylmorpholine. The reactive derivative is used in an amount of 1 to 1.5 moles per mole of the compound of formula (A), and the reaction proceeds easily at a temperature of −30 to 40°C, preferably −20 to 10°C.

The solvents may be those which do not adversely affect the reaction. Examples include, water, acetone, dioxane, acetonitrile, tetrahydrofuran, methylene chloride, chloroform, benzene, ethyl acetate and N,N-dimethylformamide. These solvents may be used as mixtures. Or those solvents which are hydrophilic may be used in combination with water.

After the reaction, the resulting product can be obtained by separating and purifying means known *per se*, such as solvent extraction, recrystallization and chromatography.

When a compound of formula (A) in which $R^1$ is a hydrogen atom is used, the reaction product obtained as above is sulfonated and then the protective group $R^2$ is eliminated. The sulfonation reaction is carried out by using a sulfonating agent such as sulfuric anhydride or a reactive derivative of sulfuric anhydride. Examples of the reactive derivative of sulfuric anhydride include sulfuric anhydride-pyridine, sulfuric anhydride-dioxane, sulfuric anhydride-trimethylamine, sulfuric anhydride-picoline, sulfuric anhydride-lutidine, and sulfuric anhydride-N,N-dimethylformamide. Instead of using such a complex formed separately, it may be formed *in situ* by using trimethylsilyl chlorosulfonate and pyridine, for example, as reagents.

In the sulfonation reaction, the sulfonating agent is used in an amount of 1 to 3 moles, preferably 1 to 2 moles, per mole of the reaction product to be sulfonated. The reaction temperature is generally 0 to 80°C, preferably 10 to 40°C. The reaction may be carried out in a solvent which does not adversely affect the reaction, such as dioxane, tetrahydrofuran, methylene chloride, pyridine and N,N-dimethylformamide either singly or in combination. After the reaction, the sulfonation product can be obtained by separating and purifying means known *per se* such as solvent extraction, recrystallization and chromatography. The sulfonation product may be obtained in the form of various sulfonate salts by using an alkali carbonate, an alkali hydroxide, an ion exchange resin, a quaternary ammonium salt, etc.

According to the process of this invention, the compound of formula (1) can be obtained by eliminating the protective group $R^2$ of the product obtained as above. The deprotection may be carried out by selecting a suitable customary method, such as methods which comprise utilizing acids, bases or hydrazine, or a reducing method depending upon the type of the protective group.

The method involving using acids is suitable for eliminating acyl groups (e.g., substituted or unsubstituted lower alkanoyl, substituted or unsubstituted lower alkoxycarbonyl, substituted or unsubstituted ar(lower)alkoxycarbonyl and lower cycloalkoxycarbonyl, substituted phenylthio groups, substituted alkylidene groups, substituted aralkylidene groups and substituted cycloalkylidene groups. Examples of acids which may be used in this method include various inorganic or organic acids such as formic acid, trifluoroacetic acid, benzenesulfonic acid, p-toluenesulfonic acid or hydrochloric acid, and cationic ion exchange resins.

The method involving using bases is applicable to the elimination of acyl groups, especially a haloalkanoyl group such as trifluoroacetyl. Examples of bases which can be used in this method include inorganic bases, for example hydroxides, carbonates and phosphates of alkali metals such as sodium and potassium or alkaline earth metals such as calcium and magnesium; and organic bases such as alkali metal acetates, trialkylamines (e.g., trimethylamine and triethylamine), picoline, N-methylpyrrolidone, and N-methylmorpholine.

When the aforesaid methods involving using acids or bases are carried out in a solvent, hydrophilic organic solvents, water, or mixtures thereof may frequently be used. When trifluoroacetic acid is used as an acid, the reaction may be promoted by adding anisole.

The method involving using hydrazine is applicable particularly to the elimination of dibasic acyl groups such as succinyl and phthaloyl.

The reducing method is applicable particularly to the elimination of acyl groups for example halo(lower)alkoxycarbonyl such as trichloroethoxycarbonyl, substituted or unsubstituted ar(lower)alkoxycarbonyl such as benzyloxycarbonyl and p-nitrobenzyloxycarbonyl, or 2-pyridylmethoxycarbonyl; and

aralkyl groups such as benzyl, benzhydryl and trityl. The reduction can be effected, for example, by using an alkali metal borohydride such as sodium borohydride, or by hydrogenation using a conventional catalyst.

Such protective groups as halo(lower)alkoxycarbonyl groups and 8-quinolyloxycarbonyl group may be eliminated by treatment with heavy metals such as copper or zinc.

The reaction temperature is not particularly restricted, and can be properly selected depending upon the chemical properties of the starting compound and the reaction product, the type of the protective group, and the type of the deprotecting method. Preferably, the reaction is carried out under mild conditions by cooling or warming.

When R is a functional derivative of a carboxyl group, it may sometimes change to a free carboxyl group during the reaction or during work-up. This case is also within the scope of the above-described method.

The pharmaceutically acceptable salt of the compound of formula (1) may be obtained as follows:

A tetra-n-butyl ammonium (TBA) salt of the compound of formula (1) may be obtained by neutralizing the compound of formula (1) with tetra-n-butylammonium hydroxide in water, a hydrophilic organic solvent, or a mixture thereof, or by dissolving the compound of formula (1) in a 0.5 M potassium phosphate, monobasic buffer, adding tetra-n-butylammonium hydrogen sulfate, and extracting the mixture with an organic solvent such as methylene chloride or chloroform. When a compound of formula (A) in which $R^1$ is $—S—SO_3^{\ominus}.T^{\oplus}BA$ acylated, the reaction mixture after the reaction may be separated and purified by silica gel chromatography to obtain the TBA salt of the compound of formula (1).

A metal salt such as a sodium or potassium salt of the compound of formula (1) may be obtained by dissolving the TBA salt of the compound of formula (1) in water, a hydrophilic organic solvent or a mixture of these, and treating the solution with a strongly acidic ion-exchange resin in a sodium or potassium form. Or it may be obtained by neutralizing the compound of formula (1) with a hydroxide or carbonate of sodium, potassium, etc.

(3S, 4R) - 3 - amino - 4 - fluoromethyl - 2 - oxoazetidine of the following formula (8) which correspond to the compound of formula (A) in which $R^1$ is a hydrogen atom may be synthesized, for example, by the method shown schematically below from L(−) - γ - fluorothreonine of formula (2) below.

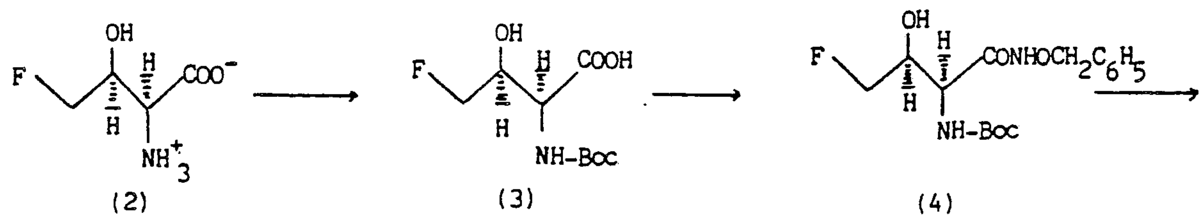

(2) (3) (4)

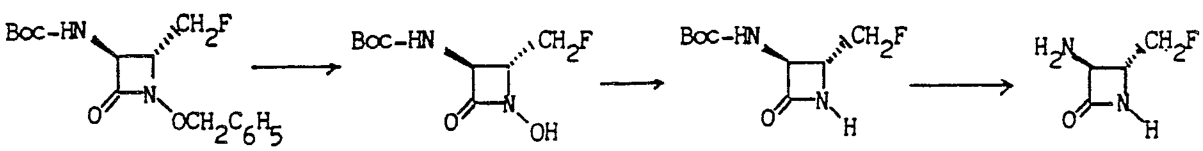

(5) (6) (7) (8)

As schematically shown above, the compound of formula (8) can be synthesized by the method of M. J. Miller et al. [J. Am. Chem. Soc., *102*, 7026 (1980)] from L(−) - γ - fluorothreonine of formula (2) [$[\alpha]_D^{20}$: $-18°$ (c = 5, $H_2O$)] as a starting material.

t-Butoxycarbonyl (to be abbreviated Boc hereinafter) - γ - fluorothreonine of formula (3) can be obtained by t-butoxycarbonylating L(−) - γ - fluorothreonine formula (2) using 2 - t - butoxycarbonylthio - 4,6 - dimethylpyrimidine (to be abbreviated Boc-S hereinafter) in, for example, dioxane. The reaction can be carried out, for example, by adding 1.5 equivalents of triethylamine to an aqueous solution of L(−) - γ - fluorothreonine (2), adding a dioxane solution containing 1.2 equivalents of Boc-S to the solution, and stirring the mixture at room temperature for 20 hours.

O - benzyl - N - Boc - γ - fluorothreonine hydroxamate (4) may be obtained by condensing the resulting N-Boc - γ - fluorothreonine (3) directly with O-benzylhydroxylamine in the presence of a condensing agent such as N,N'-dicyclohexylcarbodiimide (DCC); or by first reacting it with N-hydroxysuccinimide (HOSu) or 1-hydroxybenzotriazole (HOBt) in the presence of N,N'-dicyclohexyl carbodiimide (DCC) to convert it to an active ester, and thereafter reacting the active ester with O-benzylhydroxylamine.

The condensation reaction in the former embodiment may be carried out, for example, by adding a tetrahydrofuran solution of N - Boc - L - γ - fluorothreonine (3) to an aqueous solution of O-benzylhydroxylamine or its hydrochloride, and adding dropwise a tetrahydrofuran solution containing 1 to 3 equivalents of DCC while maintaining the pH of the solution at 3.0 to 6.0, preferably at 4.5.

The reaction in the latter embodiment may be carried out, for example, by adding 1.1 equivalents of HOSu or HOBt to a dimethylformamide solution of N - Boc - γ - fluorothreonine (3), adding 1.05

equivalents of DCC under ice cooling, stirring the mixture for 3 hours to form an active ester, adding a dimethylformamide-water solution of 1.2 equivalents of O-benzylhydroxylamine to the active ester, and reacting the mixture at 5 to 10°C for 5 hours.

The hydroxamate (4) which can be obtained as above can be converted to (3S, 4R) - (−) - 3 - Boc - amino - 1 - benzyloxy - 4 - fluoromethyl - 2 - oxoazetidine (5) [$[\alpha]_D^{20}$: −44.5° (c = 1, $CH_3OH$)] by cyclizing it in the presence of, for example, triphenylphosphine, carbon tetrachloride and triethylamine, or in the presence of, for example, diethyl azodicarboxylate (DEAD) and triphenylphosphine.

The above cyclization reaction can be carried out, for example, by dissolving O - benzyl - N - Boc - γ - fluorothreonine hydroxamate (4) in acetonitrile, adding 1.3 equivalents of triphenylphosphine and carbon tetrachloride, stirring the mixture at room temperature for 18 hours; or by adding 1.3 equivalents of triphenylphosphine to the aforesaid acetonitrile solution of the hydroxamate, adding dropwise an acetonitrile solution containing 1.3 equivalents of diethyl azodicarboxylate (DEAD) at 5°C over the course of 20 minutes and then stirring the mixture at 15°C for 4 hours.

By catalytically reducing the compound of formula (5) in the presence of a reducing catalyst such as palladium-carbon, 1-hydroxyazetidinone of formula (6) can be obtained. The reducing reaction can be carried out, for example, by dissolving (3S, 4R) - (−) - 3 - Boc - amino - 1 - benzyloxy - 4 - fluoromethyl - 2 - oxoazetidine (5) in methanol, adding 10% palladium-carbon, and hydrogenating the compound (5) under atmospheric pressure for 2 hours.

By reducing the resulting 1-hydroxyazetidinone (6) with, for example, titanium trichloride, (3S, 4R) - (−) - 3 - Boc - amino - 4 - fluoromethyl - 2 - oxoazetidine of formula (7) [$[\alpha]_D^{20}$: −110.2° (c = 1, $CH_3OH$)] can be obtained. The reducing reaction can be carried out, for example, by adding a 20% aqueous solution of titanium trichloride dropwise at 10°C over the course of 2 hours to a 50% methanol-water solution of 1-hydroxyazetidinone (6) while maintaining the pH of the methanol-water solution at 7 with 10% sodium hydroxide, and stirring the mixture for 2 hours.

By treating the compound of formula (7) with, for example, trifluoroacetic acid, (3S, 4R) - 3 - amino - 4 - fluoromethyl - 2 - oxoazetidine of formula (8) can be obtained. This reaction can be carried out, for example, by dissolving the compound of formula (7) in trifluoroacetic acid cooled at 0°C, and stirring the solution for 1 hour. The reaction may be effected by using anisole as a solvent.

A compound of formula (A) in which $R^1$ is —$SO_3H$ or an inorganic salt or an organic alkali metal salt of —$SO_3H$ can be synthesized, for example, by the method shown schematically below from the compound of formula (8) obtained as above.

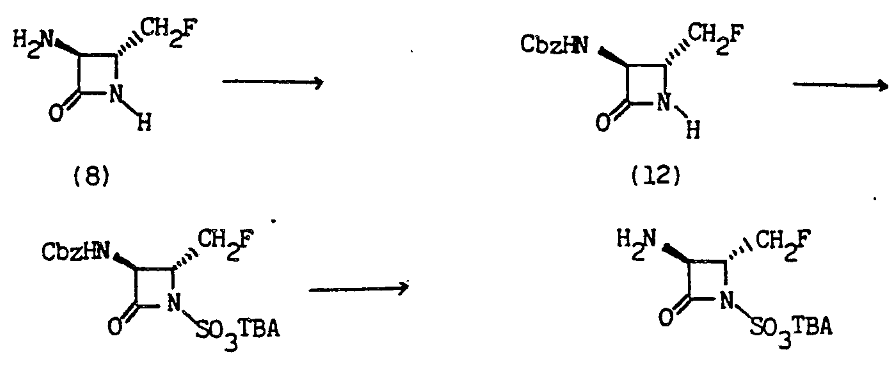

(8) (12)

(13) (14)

(3S, 4R) - 3 - amino - 4 - fluoromethyl - 2 - oxoazetidine - 1 - sulfonic acid, TBA salt of formula (14) can be obtained, as shown above schematically, by benzyloxycarbonylating the compound of formula (8) which can be obtained as above with, for example, benzyl chloroformate to obtain (3S, 4R) - 3 - benzyloxycarbonyl(Cbz for short) - amino - 4 - fluoromethyl - 2 - oxoazetidine of formula (12), sulfonating the compound of formula (12) with a sulfonating agent such as pyridine-sulfuric anhydride or 2-picoline-sulfuric anhydride complex to form (3S, 4R) - (−) - 3 - Cbz - amino - 4 - fluoromethyl - 2 - oxo - 1 - azetidinesulfonic acid, isolating it as a TBA salt of formula (13) [$[\alpha]_D^{20}$: −12.1° (c = 1, $C_2H_5OH$)], and thereafter, catalytically reducing the compound of formula (13) in the presence of, for example, palladium-carbon.

The above benzyloxycarbonylation reaction can be carried out, for example, by dissolving (3S, 4R) - 3 - amino - 4 - fluoromethyl - 2 - oxoazetidine (8) in ethyl acetate, and allowing an equivalent of benzyl chloroformate to act on the solution in the presence of triethylamine under ice cooling.

The above sulfonation reaction can be carried out, for example, by adding 2 equivalents of pyridine-sulfuric anhydride complex to a dimethylformamide solution of (3S, 4R) - 3 - Cbz - amino - 4 - fluoromethyl - 2 - oxoacetidine (12), and stirring the mixture at room temperature for 5 days.

The catalytic reduction can be carried out, for example, by adding 10% palladium-carbon to a dimethylformamide solution of (3S, 4R) - 3 - Cbz - amino - 4 - fluoromethyl - 2 - oxoazetidinesulfonic acid TBA salt (13) and hydrogenating the TBA salt (13) for 1 hour.

When the above sulfonic acid is isolated as an inorganic salt or an organic amine salt instead of isolating it as the TBA salt (13), there can be obtained a compound of formula (A) in which $R^1$ is an inorganic salt or organic amine salt of $—SO_3H$.

A compound of formula (A) in which $R^1$ is $—SO_3H$ can be obtained by eliminating the Cbz protective group for (3S, 4R) - 3 - Cbz - amino - 4 - fluoromethyl - 2 - oxoazetidinesulfonic acid, TBA salt (13) by the method described hereinabove, distilling off the reaction solvent, dissolving the residue in ethyl acetate, adding formic acid and recovering the sulfonic acid as a zwitterion compound.

The compound of formula (13) in the above scheme can be synthesized, for example in accordance with the following reaction scheme by the method of producing (3S, 4S) - 3 - Cbz - amino - 4 - methyl - 2 - oxo - 1 - azetidinesulfonic acid, TBA salt from L-threonine proposed by D. M. Floyd et al. [J. Org. Chem., *47*, 176 (1982)].

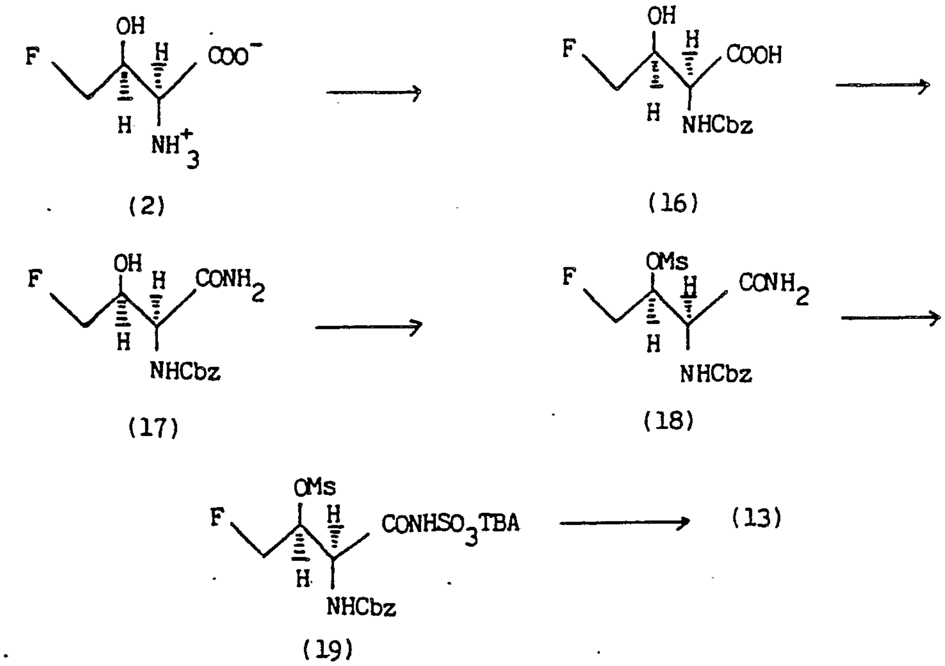

(2) (16) (17) (18) (19) (13)

As shown in the above reaction scheme, benzyl chloroformate is allowed to act on L(−) - γ - fluorothreonine (2) to convert the latter to N - Cbz - L-γ - fluorothreonine (16) which is then converted to its active ester by reacting it with N-hydroxysuccinimide (HOSu) and N,N'-dicyclohexyl carbodiimide (DCC). The active ester is reacted with ammonia to give N - Cbz - L - γ-fluorothreonineamide (17).

The reaction of the compound of formula (2) with benzyl chloroformate is carried out, for example, by adding 1.5 equivalents of triethylamine to a 50% dimethylformamide-water solution of the compound of formula (2), and adding 1.5 equivalents of benzyl chloroformate dropwise under cooling with ice.

The reaction of forming the active ester of the compound of formula (16) can be carried out, for example, by dissolving N - Cbz - L - γ - fluorothrenonine (16) and 1.1 equivalents of HOSu in tetrahydrofuran, adding 1.05 equivalents of DCC under ice cooling, and reacting the mixture at room temperature for 2 hours. The reaction of the active ester with ammonia can be carried out, for example, by adding dropwise a tetrahydrofuran solution containing 3 equivalents of ammonia under ice cooling to the active ester solution prepared as described above.

The compound of formula (17) obtained as above is reacted with methanesulfonyl chloride in, for example, pyridine, to convert it to N - Cbz - O - mesyl - L - γ - fluorothreonineamine (18) which is then sulfonated with a sulfonating agent such as pyridine-sulfuric anhydride or 2-picoline-sulfuric anhydride complex. The sulfonated product may be isolated as N - Cbz - O - mesyl - L - γ - fluorothreonine (N-sulfo) amide, TBA salt (19). The compound of formula (19) can be converted to the aforesaid intermediate (13) by cyclizing it in the presence of potassium carbonate in, for example, water/1,2-dichloroethane.

The aforesaid reaction of the compound of formula (17) with methanesulfonyl chloride can be carried out, for example, by dissolving the compound of formula (17) in anhydrous pyridine, adding 1.17 equivalents of methanesulfonyl chloride dropwise at less than 5°C., and stirring the mixture for 2 hours at less than 5°C.

The sulfonation of the resulting compound of formula (18) can be carried out, for example, by adding a methylene chloride solution of 4 equivalents of 2-picoline/chlorosulfonic acid complex dropwise to a methylene chloride suspension of the compound of formula (18), and boiling the mixture under reflux for 16 hours.

The cyclization reaction of the compound of formula (19) may be carried out, for example, by adding

dropwise a 1,2-dichloroethane solution of the compound of formula (19) to a mixture, being boiled under reflux, of an aqueous solution of potassium carbonate and 1,2-dichloroethane.

According to this invention, there can be provided an antibacterial composition composed of an antibacterially effective amount of an optically active compound represented by the following formula (1)

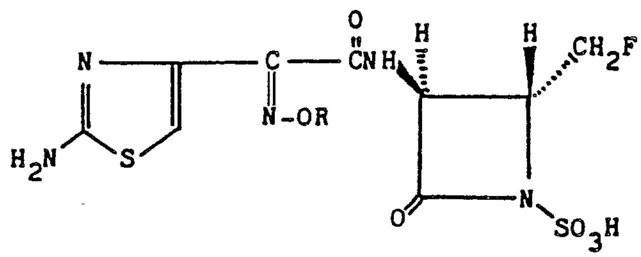

...(1)

wherein R represents a group selected from the class consisting of linear or branched $C_1$—$C_4$ groups, $C_3$—$C_4$ alkenyl groups, $C_3$—$C_4$ alkynyl groups (the latter being optionally substituted by carboxy), a cyanomethyl group, $C_3$—$C_6$ cycloalkyl groups which may be 1-carboxy-substituted, 1-carboxy-1-methyl-$C_2$—$C_4$ lower alkyl groups, a benzyl group and a phenyl group,
or a pharmaceutically acceptable salt thereof, and a pharmaceutically acceptable diluent or carrier.

The compound of formula (1) in accordance with this invention may be used as such or as its pharmaceutically acceptable salt for the prevention or treatment of bacterial infections. The compound of this invention or its salt, either alone or in combination with a pharmaceutically acceptable diluent or carrier, can be administered orally or parenterally in forms suitable for administration.

Examples of the pharmaceutically acceptable liquid or solid diluents or carriers which can be used in this invention are shown below.

For tablets and capsules, there can usually be employed gum arabic, gelatin, sorbitol, tragacanth, polyvinylpyrrolidone, lactose, sucrose, corn starch, calcium phosphate, glysine, magnesium stearate, talc, polyethylene glycol, silica, sodium laurylsulfate, etc.

For liquid preparations, there can usually be employed sorbitol syrup, methyl cellulose, glucose, sucrose syrup, gelatin, hydroxyethyl cellulose, carboxymethyl cellulose, aluminum stearate gel, edible oils, lecithin, sorbitan monooleate, gum arabic, almond oil, coconut oil, oily esters, propylene glycol, ethyl alcohol, methyl p-hydroxybenzoate, propionic acid, and sorbic acid.

Suppositories may contain ordinary suppository bases such as cacao butter and glycerides.

The antibacterial composition of this invention may be in various forms, for example orally administrable forms such as capsules, tablets, granules, liquid preparations, suspensions, emulsions, syrups or elixirs; and parenterally administrable forms, for example injections, suppositories, and external or local agents such as oil ointments, creams, lotions, coating agents, powders and drops. Means for preparing these various formulations are known, and can be utilized in this invention.

The antibacterial composition of this invention contains an antibacterially effective amount of the compound of formula (1) or a pharmaceutically acceptable salt thereof. The content of the compound of formula (1) or its salt is, for example, about 0.1 to about 99% by weight based on the weight of the composition.

The dosage of the compound of formula (1) or its salt can be varied properly depending upon the condition of a patient, the type of the bacterium to be controlled, etc. An average single dose of about 50 mg, 100 mg, 250 mg or 500 mg has been found to be effective for the treatment of infections induced by many pathogenic bacteria. Generally, the dose is 1 mg to 1000 mg or more per day.

The antibacterial composition of this invention is effective for the treatment and prevention of various bacterial infections caused by a variety of bacteria. Examples of the infections include hemasosepsis, bronchitis, pneumonia, pulmonary abscess, peritonitis, nephropyelitis, cystitis, urethritis, cholangitis, cholecystitis, infections in the uterus (e.g., endometritis and myometritis), and secondary infections after wounds, burns and surgical operations, which are caused by Gram-negative bacteria, such as E. coli, Klebsiella, Serratia, Enterobacter, Salmonella, Pseudomonas and Proteus.

Table I below summarizes the antibacterial activities (MIC) of some typical examples of the active compounds of this invention (compounds A to G).

Compound A: Potassium (3S, 4R) - (—) - 3[(Z) - 2 - (2 - amino - 4 - thiazolyl) - 2 - methoxyiminoacetamide] - 4 - fluoromethyl - 2 - oxo - 1 - azetidinesulfonate

Compound B: Potassium (3S, 4R) - (—) - 3 - [(Z) - 2 - (2 - amino - 4 - thiazolyl) - 2 - ethoxyiminoacetamide] - 4 - fluoromethyl - 2 - oxo - 1 - azetidinesulfonate

Compound C: Potassium (3S, 4R) - (—) - 3 - [(Z) - 2 - (2 - amino - 4 - thiazolyl) - 2 - carboxymethoxyiminoacetamide] - 4 - fluoromethyl - 2 - oxo - 1 - azetidinesulfonate

Compound D: Potassium (3S, 4R) - (—) - 3 - [(Z) - 2 - (2 - amino - 4 - thiazolyl) - 2 - (1 - carboxy - 1 - methylethoxyimino)acetamide] - 5 - fluoromethyl - 2 - oxo - 1 - azetidinesulfonate

Compound E: Potassium (3S, 4R) - (—) - 3 - [(Z) - 2 - (2 - amino - 4 - thiazolyl) - 2 - benzyloxyiminoacetamide] - 4 - fluoromethyl - 2 - oxo - 1 - azetidinesulfonate

Compound F: Potassium (3S, 4R) - (−) - 3 - [(Z) - 2 - (2 - amino - 4 - thiazolyl) - 2 - (1 - carboxyl - 1 - cyclobutoxyimino)acetamide] - 4 - fluoromethyl - 2 - oxo - 1 - azetidinesulfonate

Compound G: Potassium (3S, 4R) - (−) - 3 - [(Z) - 2 - (2 - amino - 4 - thiazolyl) - 2 - (1 - carboxy - 1 - cyclopentoxyimino)acetamide] - 4 - fluoromethyl - 2 - oxo - 1 - azetidinesulfate.

TABLE I

MIC (Number of cells $10^6$ CFU/ml, nutrient agar)

| Test bacterium | Compound | | | | | | |
|---|---|---|---|---|---|---|---|
| | A | B | C | D | E | F | G |
| Escherichia coli NIHJ | < 0.05 | < 0.05 | 0.2 | < 0.05 | 0.2 | < 0.05 | < 0.05 |
| Klebsiella pneumoniae D–11 | < 0.05 | < 0.05 | 0.2 | < 0.05 | – | < 0.05 | < 0.05 |
| Serratia marcescens IAM–1223 | 0.1 | < 0.05 | 0.1 | < 0.05 | 0.78 | < 0.05 | < 0.05 |
| Enterobacter aerogenes IMA–12348 | 0.1 | 0.1 | – | 0.1 | 6.25 | < 0.05 | 0.1 |
| Salmonella paratyphi A–1015 | < 0.05 | < 0.05 | < 0.05 | < 0.05 | < 0.05 | < 0.05 | < 0.05 |
| Pseudomonas aeruginosa D–15 | > 25 | > 25 | > 25 | 12.5 | > 25 | 6.25 | 6.25 |
| Pseudomonas aeruginosa A–9930 | > 25 | > 25 | > 25 | 6.25 | > 25 | 1.56 | 1.56 |
| Acinetobacter calcoa IAM–12087 | 12.5 | 25 | > 25 | 25 | > 25 | > 25 | > 25 |
| Proteus vulgaris A–9438 | 0.1 | < 0.05 | 0.1 | < 0.05 | 0.1 | < 0.05 | < 0.05 |
| Proteus mirabilis A–9554 | < 0.05 | < 0.05 | < 0.05 | < 0.05 | 6.25 | < 0.05 | < 0.05 |
| Proteus morganii A–9553 | 6.25 | 12.5 | 6.25 | 12.5 | 25 | 25 | 12.5 |
| Alcaligenes faecalis ATCC–8750 | 12.5 | 12.5 | > 25 | > 25 | > 25 | > 25 | > 25 |
| Staphylococcus aureus 209–P | > 25 | 25 | > 25 | > 25 | 6.25 | > 25 | > 25 |
| Staphylococcus epidermidis IAM–12012 | 25 | 6.25 | > 25 | > 25 | 1.56 | – | – |
| Sarcina lutea PCI–1001 | 6.25 | 1.56 | > 25 | 12.5 | 0.39 | 25 | 12.5 |
| Bacillus subtilis PCI–219 | 25 | 6.25 | 25 | > 25 | 3.13 | 25 | 12.5 |

The following Examples illustrate the production of the compounds of this invention.

Example 1

O-Benzyl-N-Boc-γ-fluorothreonine hydroxamate:—

(A) One gram (7.29 millimoles) of L - (−) - γ - fluorothreonine and 1.5 ml (10.9 mmoles) of triethylamine were dissolved in 4 ml of water, and a solution of 1.9 g (8.02 mmoles) of Boc-S in 4 ml of dioxane was added to the solution, and the mixture was stirred at room temperature for about 20 hours. Water (11 ml) was added to this reaction solution, and the mixture was washed with two 15 ml portions of ethyl acetate. Then, the aqueous layer was treated with 11 ml of ethyl acetate, and the mixture was adjusted to pH 2 with 6N HCl. The ethyl acetate layer was separated and further extracted with two 6 ml portions of ethyl acetate. The extracts were combined, washed with two 7 ml portions of 5% HCl saturated with sodium chloride, dried over anhydrous sodium sulfate, and evaporated to dryness under reduced pressure to give 1.93 g of N - Boc - L - γ - fluorothreonine as an oil.

(B) 2.32 g (14.6 mmoles) of O-benzylhydroxylamine hydrochloride was dissolved in 64 ml of water, and the solution was adjusted to pH 4.5 with 6N NaOH. To the solution was added a solution of 1.93 g of N - Boc - L - γ - fluorothreonine in 16 ml of tetrahydrofuran (THF). Then, while maintaining the pH of the solution at 4.5 with 6N hydrochloric acid, a solution of 3 g (14.6 mmoles) of N,N'-dicyclohexylcarbodiimide (DCC) in 48 ml of THF was added dropwise with stirring. The stirring was continued until there was no variation in pH. Then, THF was evaporated under reduced pressure, and 80 ml of ethyl acetate was added. Insoluble N,N'-dicyclohexylurea was removed by filtration, and the ethyl acetate layer was separated. The ethyl acetate layer was further extracted with two 40 ml portions of ethyl acetate. The ethyl acetate extracts were combined, dehydrated with sodium sulfate, and concentrated under reduced pressure. The precipitated crystals were collected by filtration, and washed with ether. Yield 1.95 g (yield from L - γ - fluorothreonine: 78.1%).

Mp: 122—123°C.

IR (KBr): $cm^{-1}$

3350, 1665, 1530, 1370, 1310, 1250, 1170.

NMR (DMSO-$d_6$): δ in ppm

1.43 (9H, s), 3.9—4.7 (4H, m), 4.83 (2H, s), 5.46 (1H, d, J=5Hz), 6.45 (1H, d, J=7.5Hz), 7.43 (5H, s), 11.28 (1H, s).

Example 2

(3S, 4R)-(−)-3-Boc-amino-1-benzyloxy-4-fluoromethyl-2-oxoazetidine:—

(A) 1.46 g (4.26 mmoles) of o - benzyl - N - Boc - γ - fluorothreonine hydroxamate was dissolved in 42 ml of anhydrous acetonitrile, and 1.55 g (5.54 mmoles) of triphenylphosphine, 0.54 ml (5.54 mmoles) of carbon tetrachloride and 0.54 ml (6.4 mmoles) of triethylamine were added. The mixture was stirred at room temperature for about 18 hours in an atmosphere of nitrogen. The reaction mixture was concentrated under reduced pressure, and chromatographed on a silica gel flash column using hexane-ethyl acetate (4:1) as an eluent. The product was concentrated, and the residue was crystallized from isopropyl ether (IPE) to give 390 mg (28.3%) of the title compound.

Mp: 86—87°C.

$[\alpha]_D^{20}$: −44.5° (c = 1, $CH_3OH$)

Elemental analysis for $C_{16}H_{21}FN_2O_4$

| | | | |
|---|---|---|---|
| Calculated: | C, 59.25; | H, 6.53; | N, 8.64 |
| Found: | C, 59.32; | H, 6.75; | N, 8.70 |

IR (KBr): $cm^{-1}$

3330, 1760, 1710, 1540, 1285, 1170, 995.

NMR (DMSO-$d_6$): δ in ppm

1.41 (9H, s), 3.9—4.4 (2H, m), 47 (2H, dd, J=48 & 3Hz), 4.99 (2H, s), 7.48 (5H, s), 7.67 (1H, d, J=7Hz).

(B) 17.15 g (50.1 mmoles) of O - benzyl - N - Boc - γ - fluorothreonine hydroxamate and 21 g (65.1 mmoles) of triphenylphosphine were dissolved in 430 ml of anhydrous acetonitrile. While maintaining the solution at 5°C, a solution of 9.47 ml (65.1 mmoles) of diethyl azodicarboxylate in 20 ml of anhydrous acetonitrile was added dropwise over the course of 20 minutes. The reaction mixture was stirred at 15°C for 4 hours, and concentrated under reduced pressure, separated by silica gel flash column chromatography, and crystallized from IPE to give 6.78 g (41.7%) of the title compound.

Example 3

(3S, 4R)-(−)-3-Boc-amino-4-fluoromethyl-2-oxoazetidine:—

(A) Ten grams (30.8 mmoles) of (3S, 4R) - (−) - 3 - Boc - amino - 1 - benzyloxy - 4 - fluoromethyl - 2 - oxoazetidine was dissolved in 500 ml of methanol, and 1 g of 10% palladium-carbon was added to the solution. The oxoazetidine compound was hydrogenated at atmospheric pressure for 2 hours. The catalyst was separated by filtration, and the filtrate was evaporated to give (3S, 4R) - (−) - 3 - Boc - amino - 4 - fluoromethyl - 1 - hydroxy - 2 - oxoazetidine.

(B) A solution of the 1-hydroxyazetidinone obtained in Example 3, (A) above in 150 ml of methanol and 150 ml of water was adjusted to pH 7 with 10% NaOH. While maintaining the pH of this solution at 7, a 20% aqueous solution of titanium trichloride was added dropwise at 10°C over the course of 2 hours, and the mixture was further stirred for 2 hours. The reaction mixture was adjusted to pH 8, and 600 ml of an 8% NaCl solution and 1200 ml of ethyl acetate were added. The insoluble material was removed by filtration, and the organic layer was separated from the aqueous layer. The aqueous layer was further extracted with 600 ml of ethyl acetate. The extracts were combined, dried over sodium sulfate, and concentrated under reduced pressure. The residue was crystallized from IPE to give 1.82 g (yield from the 1-benzyloxy compound: 27.1%) of the title compound.

Mp: 189—190°C (dec.).

$[\alpha]_D^{20}$: −110.2° (c = 1, $CH_3OH$)

Elemental analysis for $C_9H_{15}FN_2O_3$

| | | | |
|---|---|---|---|
| Calculated: | C, 49.54; | H, 6.92; | N, 12.84 |
| Found: | C, 48.99; | H, 7.00; | N, 12.56 |

IR (KBr): $cm^{-1}$

3270, 1760, 1750, 1685, 1540, 1295, 1170, 1000.

NMR (DMSO-$d_6$): δ in ppm

1.39 (9H, s), 3.3—4.0 (1H, m), 4.2—4.9 (3H, m), 7.56 (1H, d, J=7Hz), 8.2 (1H, brs).

## Example 4

(3S, 4R)-(−)-3-[(Z)-2-(2-amino-4-thiazolyl)-2-methoxyiminoacetamide]-4-fluoromethyl-2-oxo-1-azetidinesulfonic acid:—

(A) 610 mg (1.37 mmoles) of (Z) - 2 - (2 - tritylamino - 4 - thiazolyl) - 2 - methoxyiminoacetic acid was added under cooling at −10°C to a solution of 315 mg (1.51 mmoles) of phosphorus pentachloride in 15 ml of methylene chloride. The reaction mixture was stirred at −5°C for 30 minutes, and cooled to −10°C. Then 0.42 ml (3.02 mmoles) of triethylamine and 5 ml of water were added, and the mixture was stirred at 0°C for 3 minutes. The organic layer was taken and dried over anhydrous potassium carbonate-sodium sulfate to prepare an acid chloride solution.

Separately, a solution of 300 mg (1.73 mmoles) of (3S, 4R) - (−) - 3 - Boc - amino - 4 - fluoromethyl - 2 - oxoazetidine in 1.5 ml of cold trifluoroacetic acid was stirred for 1 hour under ice cooling, and evaporated under reduced pressure. Furthermore, 5 ml of ethyl acetate was added, and the mixture was evaporated. To the residue was added 20 ml of ethyl acetate, and the mixture was cooled to −10°C. Triethylamine (0.96 ml; 6.88 mmoles) was added, and the acid chloride solution prepared in advance was added dropwise over the course of 10 minutes. The mixture was stirred at −10 to 0°C for 30 minutes, and after removing the cooling bath, further stirred for 30 minutes. The reaction mixture was concentrated under reduced pressure. The residue was extracted by adding ethyl acetate and water. The organic layer was taken, dried over sodium sulfate, and concentrated to dryness under reduced pressure. The residue was chromatographed on a silica gel flash column using chloroform-methanol (97:3) as an eluent. Fractions containing the desired compound were concentrated under reduced pressure, and the precipitate was collected by filtration using ether. There was obtained 600 mg (80.3%) of (3S, 4R) - (−) - 3 - [(Z) - 2 - (2 - tritylamino - 4 - thiazolyl) - 2 - methoxyiminoacetamide) - 4 - fluoromethyl - 2 - oxoazetidine.

(B) Pyridine-sulfuric anhydride complex (525 mg; 3.3 mmoles) was added to a solution of 600 mg (1.1 mmoles) of (3S, 4R) - (−) - 3 - [(Z) - 2 - (2 - tritylamino - 4 - thiazolyl) - 2 - methoxyiminoacetamide] - 4 - fluoromethyl - 2 - oxoazetidine in 10 ml of anhydrous dimethylformamide (DMF), and the mixture was stirred at room temperature for 3 days. DMF was then evaporated under reduced pressure, and the residue was partitioned between ethyl acetate and water, and the organic layer was collected. The organic layer was dried over sodium sulfate, and then chromatographed on a silica gel flash column using chloroform-methanol (97:3) as an eluent. Fractions containing the desired product were concentrated under reduced pressure to give (3S, 4R) - (−) - 3 - [(Z) - 2 - (2 - tritylamino - 4 - thiazolyl) - 2 - methoxyiminoacetamide] - 4 - fluoromethyl - 2 - oxoazetidine - 1 - sulfonic acid.

(C) The resulting (3S, 4R) - (−) - 3 - [(Z) - 2 - (2 - triylamino - 4 - thiazolyl) - 2 - methoxyiminoacetamide] - 4 - fluoromethyl - 2 - oxo - 1 - azetidinesulfonic acid was dissolved in 10 ml of formic acid, and left to stand at 5°C for 2 days. The solvent was removed from the reaction mixture under reduced pressure, the residue was triturated with acetone, and collected as a powder by filtration. The powder was chromatographed on a silica gel flash column using chloroform-methanol (6:4) as an eluent to give 65 mg (the yield through the two steps, sulfonation and de-tritylation: 15.4%) of the title compound.

IR (KBr): $cm^{-1}$

3450, 1770, 1665, 1625, 1530, 1270, 1240, 1050.

## Example 5

(3S, 4R)-(−)-3-[(Z)-2-(2-amino-4-thiazolyl)-2-(1-carboxy-1-methylethoxyimino)acetamide]-4-fluoromethyl-2-oxo-1-azetidinesulfonic acid:—

(A) 784 mg (1.37 mmoles) of (Z) - 2 - (2 - tritylamino - 4 - thiazolyl) - 2 - (1 - t - butoxycarbonyl - 1 -

methylethoxyimino)acetic acid was added at −10°C to a solution of 315 mg (1.51 mmoles) of phosphorus pentachloride in 15 ml of methylene chloride. The reaction mixture was stirred at −5°C for 30 minutes, cooled to −10°C, and after adding 5 ml of water and 0.42 ml (3.02 mmoles) of triethylamine, stirred at 0°C for 3 minutes. The organic layer was dried over anhydrous potassium carbonate-sodium sulfate to prepare an acid chloride solution.

Separately, a solution of 300 mg (1.37 mmoles) of (3S, 4R) - (−) - Boc - amino - 4 - fluoromethyl - 2 - oxoazetidine in 1.5 ml of cold trifluoroacetic acid was stirred under ice cooling for 1 hour. The reaction mixture was concentrated under reduced pressure, and 5 ml of ethyl acetate was added to the residue. The mixture was again concentrated. Ethyl acetate (20 ml) was added to the residue, and 0.96 ml (6.88 mmoles) of triethylamine was added at −10°C, and then the acid chloride solution prepared as above was added dropwise over the course of 10 minutes. The mixture was stirred at −10 to 0°C for 30 minutes and then at room temperature for 30 minutes. The reaction mixture was concentrated under reduced pressure. The residue was extracted by adding ethyl acetate and water. The organic layer was taken, dried over sodium sulfate and concentrated under reduced pressure. The residue was purified by silica gel flash column chromatography using chloroform-methanol (97:3) as an eluent to give 340 mg (36.8%) of (3S, 4R) - (−) - 3 - [(Z) - 2 - (2 - tritylamino - 4 - thiazolyl) - 2 - (1 - t - butoxycarbonyl - 1 - methylethoxyimino)acetamide] - 4 - fluoromethyl - 2 - oxoazetidine.

IR (KBr): $cm^{-1}$

3400, 1770, 1740, 1670, 1590, 1570, 1200, 1190, 1040, 995.

(B) A pyridine-sulfuric anhydride complex (240 mg; 1.5 mmoles) was added to a solution of 340 mg (0.51 mmole) of the azetidinone obtained in Example 5, (A) in 6 ml of anhydrous DMF, and the mixture was stirred at room temperature for 3 days. The reaction mixture was concentrated under reduced pressure and extracted by adding ethyl acetate and water. The organic layer was separated, dried over sodium sulfate, and concentrated to dryness under reduced pressure. The residue was chromatographed on a silica gel flash column using chloroform-methanol as an eluent to give 180 mg (47.3%) of (3S, 4R) - (−) - 3 - [(Z) - 2 - (2 - tritylamino - 4 - thiazolyl) - 2 - (1 - t - butoxycarbonyl - 1 - methylethoxyimino)acetamide] - 4 - fluoromethyl - 2 - oxo - 1 - azetidinesulfonic acid.

IR (KBr): $cm^{-1}$

3400, 1755, 1725, 1670, 1590, 1570, 1280, 1245, 1200, 1140, 1045.

(C) A solution of the compound obtained in Example 5, (B) (180 mg; 0.24 mmole) in 5 ml of formic acid was left to stand at 5°C for 4 days, and concentrated. The residue was triturated with acetone, and collected as a powder by filtration. The solid was chromatographed on a silica gel flash column chromatography using chloroform-methanol (7:3) as an eluent. Fractions containing the desired product were concentrated under reduced pressure. The residue was collected by filtration as a powder using acetone to give 60 mg (55.3%) of the title compound.

IR (KBr): $cm^{-1}$

1775, 1665, 1530, 1535, 1400, 1270, 1240, 1200, 1165, 1050.

## Example 6

(3S, 4R)-(−)-3-Cbz-amino-4-fluoromethyl-2-oxoazetidine:—

3.0 g (13.7 mmoles) of (3S, 4R) - (−) - 3 - Boc - amino - 4 - fluoromethyl - 2 - oxoazetidine was dissolved in 15 ml of trifluoroacetic acid cooled to 0°C. The solution was stirred at the same temperature for 1 hour. The reaction mixture was evaporated under reduced pressure, and benzene was added to the residue. The mixture was evaporated. This operation was repeated twice. The residue was dissolved in 100 ml of ethyl acetate, and the solution was cooled to 0°C. Triethylamine (5 ml) was added, and 2 ml (13.7 mmoles) of benzyl chloroformate was added dropwise with stirring. The solution was stirred for 2 hours, and then cold water was added. The ethyl acetate layer was collected, dehydrated over sodium sulfate, and concentrated under reduced pressure. The residue was purified by silica gel flash column chromatography using ethyl acetate-hexane (7:3) as an eluent and crystallized from isopropyl ether to give 1.8 g (52%) of the title compound.

Mp: 98—100°C.

IR (KBr): $cm^{-1}$

3280, 1760, 1745, 1690, 1545, 1270, 1145, 1020, 1000.

NMR (DMSO-$d_6$): δ in ppm

3.7—4.1 (1H, m), 4.2—4.9 (3H, m), 5.11 (2H, s), 5.96 (1H, d, J=8Hz), 6.6 (1H, s), 7.36 (5H, s).

## Example 7

Tetra-n-butylammonium (3S, 4R)-(−)-3-Cbz-amino-4-fluoromethyl-2-oxo-1-azetidinesulfonate:—

Pyridine-sulfuric anhydride complex (1.91 g; 12 mmoles) was added to a solution of 1.51 g (6 mmoles) of (3S, 4R)-(−)-3-Cbz-amino-4-fluoromethyl-2-oxoazetidine in 20 ml of dimethylformamide (DMF). The mixture was stirred at room temperature for 5 days. The reaction mixture was poured into 300 ml of a cold 0.5 M potassium phosphate, monobasic solution adjusted to pH 5.5, and washed with three 100 ml portions of methylene chloride, and 2.04 g (0.6 mmole) of tetra-n-butylammonium hydrogen sulfate was added. The aqueous solution was extracted with four 100 ml portions of methylene chloride. The extracts were washed with 8% sodium chloride solution, dehydrated over sodium sulfate, and concentrated. The

precipitated crystals were collected by filtration after adding ethyl acetate. Thus, 2.5 g (73%) of the title compound was obtained.

Mp: 113—115°C.

$[\alpha]_D^{20}$: −12.1° (c = 1, $C_2H_5OH$)

Elemental analysis for $C_{28}H_{48}N_3FO_6S$

| | | | |
|---|---|---|---|
| Calculated: | C, 58.61; | H, 8.43; | N, 7.32 |
| Found: | C, 58.43; | H, 8.79; | N, 7.40 |

IR (KBr): $cm^{-1}$

1765, 1720, 1530, 1280, 1135, 1040.

NMR (DMSO-$d_6$): δ in ppm

0.95 (12H, t, J=6.5Hz), 1.10—1.80 (16H, m), 3.05—3.40 (8H, m), 3.93 (1H, m), 4.53 (1H, d, J=8.7Hz), 4.72 (2H, dd, J=42 & 2Hz), 5.08 2H, s), 7.40 (5H, s), 8.05 (1H, d, J=8.7Hz).

## Example 8

Potassium (3S, 4R)-(−)-3-[(Z)-2-(2-amino-4-thiazolyl)-2-methoxyiminoacetamide]-4-fluoromethyl-2-oxo-1-azetidinesulfonate:—

(A) 350 mg of 10% Palladium carbon was added to a solution of 700 mg (1.2 mmoles) of tetra-n-butyl ammonium (3S, 4R) - (−) - 3 - Cbz - amino - 4 - fluoromethyl - 2 - oxo - 1 - azetidinesulfonate in 15 ml of dimethylformamide (DMF), and the azetidine compound was hydrogenated for 1 hour. The catalyst was removed by filtration through Celite, and washed with 2 ml of DMF. The filtrate and the washing were combined, and 178 mg (1.3 mmoles) of 1-hydroxybenzotriazole (HOBt) and 241 mg (1.2 mmoles) of (Z) - 2 - (2 - amino - 4 - thiazolyl) - 2 - methoxyiminoacetic acid were added. With ice cooling, 260 mg (1.26 mmoles) of N,N'-dicyclohexylcarbodiimide (DCC) was added, and the mixture was stirred at room temperature for 17 hours. The filtrate was separated by filtration, and the filtrate was distilled off under reduced pressure. The residue was purified by silica gel chromatography using ethyl acetate-acetone (4:1—1:2) to give 524 mg (70%) of tetra-n-butylammonium (3S, 4R) - (−) - 3 - [(Z) - 2 - (2 - amino - 4 - thiazolyl) - 2 - methoxyiminoacetamide] - 4 - fluoromethyl - 1 - azetidinesulfonate.

IR (KBr): $cm^{-1}$

1770, 1670, 1620, 1535, 1270, 1040.

NMR (DMSO-$d_6$): δ in ppm

0.95 (12H, t, J=7Hz), 1.10—1.80 (16H, m), 3.00—3.50 (8H, m), 3.70—4.10 (4H, m), 4.76 (2H, m), 4.82 (1H, dd, J=7.5 & 2.0Hz), 6.76 (1H, s), 7.21 (2H, br s), 9.36 (1H, d, J=7.5Hz).

(B) A solution of 479 mg (0.77 mmole) of the tetra-n-butyl ammonium salt obtained in Example 8, (A) in 20 ml of water was treated with activated carbon, and passed through a column filled with 5 ml of Diaion SK—102 ($K^+$). Fractions containing the desired product were lyophilized to give 260 mg (87.2%) of the title compound.

$[\alpha]_D^{20}$: −23.5° (c = 1, $H_2O$)

IR (KBr): $cm^{-1}$

1775, 1665, 1620, 1535, 1380, 1270, 1245, 1050, 960, 815, 720, 650.

NMR ($D_2O$): δ in ppm

4.03 (3H, s), 4.15 (1H, m), 4.92 (2H, m), 5.01 (1H, d, J=2.5Hz), 6.97 (1H, s).

## Example 9

Potassium (3S, 4R)-(−)-3-[(Z)-2-(2-amino-4-thiazolyl)-2-(1-carboxy-1-methylethoxyimino)acetamide]-4-fluoromethyl-2-oxo-1-azetidinesulfonate:—

(A) A solution of 2.14 g (3.73 mmoles) of tetra-n-butylammonium (3S, 4R) - (−) - 3 - Cbz - amino - 4 - fluoromethyl - 2 - oxo - 1 - azetidinesulfonate in 75 ml of DMF was subjected to hydrogenation to 2 hours in the presence of 640 mg of 10% palladium carbon. The catalyst was separated by filtration. To the filtrate were added 1.64 g (3.73 mmoles) of 2 - (2 - amino - 4 - thiazolyl) - 2 - (1 - diphenylmethoxycarbonyl - 1 - methylethoxyimino)acetic acid, 0.55 g (4.07 mmoles) of 1-hydroxybenzotriazole (HOBt), and 0.77 g (3.75 mmoles) of N,N'-dicyclohexylcarbodiimide and the mixture was stirred at room temperature for 20 hours. The reaction mixture was concentrated under reduced pressure to remove DMF. Methylene chloride was added to the residue, and the insoluble material was removed by filtration. The residue was subjected to silica gel flash column chromatography using acetone-methylene chloride (3:7 → 6:4). The resulting solid was mixed with 9.4 ml of anisole, and the mixture was cooled to −15°C. Trifluoroacetic acid (47 ml) was added, and the mixture was stirred at 0°C for 15 minutes. Ethyl acetate was added to the residue, and it was collected by filtration. The collected product was crystallized from methanol-ethyl acetate to give 1.20 g (70%) of (3S, 4R) - (−) - 3 - [2 - (2 - amino - 4 - thiazolyl) - 2 - (1 - carboxy - 1 - methylethoxyimino)acetamide] - 4 - fluoromethyl - 2 - oxo - 1 - azetidinesulfonic acid.

(B) 720 mg of the compound obtained in Example 9, (A) was suspended in 35 ml of water, and under cooling, dissolved with its pH adjusted to 5.5 with 0.4N potassium hydroxide. The solution was lyophilized.

Acetonitrile was added to the residue, and the mixture was concentrated three times. The residue was triturated with ether and collected by filtration to give 840 mg of the title compound.

$[\alpha]_D^{20}$: −16.4° (c = 1, $H_2O$)

IR (KBr): $cm^{-1}$

3400, 1780, 1670, 1590, 1540, 1400, 1370, 1275, 1245, 1250, 1160, 1050.

NMR (DMSO-$d_6$): δ in ppm

1.40 (3H, s), 1.44 (3H, s), 3.8—4.3 (1H, m), 4.3—5.2 (3H, m), 6.80 (1H, s), 7.23 (2H, br s).

Example 10

Potassium (3S, 4R)-(−)-3-[(Z)-2-(2-amino-4-thiazolyl)-2-phenoxyiminoacetamide]-4-fluoromethyl-2-oxo-1-azetidinesulfonate:—

170 mg of 10% palladium carbon was added to a solution of 574 mg (1 mmole) of tetra-n-butylammonium (3S, 4R) - (−) - 3 - Cbz - amino - 4 - fluoromethyl - 2 - oxo - 1 - azetidinesulfonate in 20 ml of DMF, and the tetra-n-butylammonium salt was hydrogenated at room temperature for 2 hours. The catalyst was separated by filtration, and to the filtrate were added 263 mg (1 mmole) of (Z) - 2 - (2 - amino - 4 - thiazolyl) - 2 - phenoxyiminoacetic acid, 148 mg (1.1 mmoles) of 1-hydroxybenzotriazole (HOBt) and 206 mg (1 mmole) of N,N'-dicyclohexylcarbodiimide (DCC). The mixture was stirred at room temperature for 18 hours. The reaction mixture was evaporated under reduced pressure, and methylene chloride was added to the residue. The insoluble material was separated by filtration. The filtrate was subjected to silica gel flash column chromatography using acetone-methylene chloride (3:7—6:4) as an eluent. Fractions containing the desired product were collected and concentrated to dryness under reduced pressure. The residue was dissolved in water, and passed through a column of Diaion SK—102 ($K^+$). Fractions containing the desired product were lyophilized to give 340 mg (70.6%) of the title compound as a white powder.

$[\alpha]_D^{20}$: −16.9° (c = 1, $H_2O$)

IR (KBr): $cm^{-1}$

3470, 3370, 1780, 1670, 1620, 1595, 1540, 1490, 1280, 1250, 1200, 1055.

NMR (DMSO-$d_6$): δ in ppm

3.8—4.2 (1H, m), 4.4—5.2 (3H, m), 7.0—7.5 (8H, m), 9.63 (1H, d, J=8Hz).

Example 11

Potassium (3S, 4R)-(−)-3-[(Z)-2-(2-amino-4-thiazolyl)-2-ethoxyiminoacetamide]-4-fluoromethyl-2-oxo-1-azetidinesulfonate:—

Substantially in accordance with the method of Example 10, 200 mg (46.1%) of the title compound was obtained by using (Z) - 2 - (2 - amino - 4 - thiazolyl) - 2 - ethoxyiminoacetic acid.

$[\alpha]_D^{20}$: −26° (c = 1, $H_2O$)

IR (KBr): $cm^{-1}$

3450, 1775, 1660, 1620, 1535, 1270, 1240, 1050.

NMR (DMSO-$d_6$): δ in ppm

1.21 (3H, t, J=7Hz), 3.8—4.2 (1H, m), 4.21 (2H, q, J=7Hz), 4.3—5.2 (3H, m), 6.74 (1H, s), 7.20 (2H, br s), 9.30 (1H, d, J=8Hz).

Example 12

Potassium (3S, 4R)-(−)-3-[(Z)-2-(2-amino-4-thiazolyl)-2-benzyloxyiminoacetamide]-4-fluoromethyl-2-oxo-1-azetidinesulfonate:—

Substantially in accordance with the method of Example 10, 420 mg (84.8 g) of the title compound was obtained by using (Z) - 2 - (2 - amino - 4 - thiazolyl) - 2 - benzyloxyiminoacetic acid.

$[\alpha]_D^{20}$: −16° (c = 1, $H_2O$)

IR (KBr): $cm^{-1}$

3450, 1775, 1670, 1620, 1535, 1270, 1245, 1050.

NMR (DMSO-$d_6$): δ in ppm

3.8—4.2 (1H, m), 4.3—5.1 (3H, m), 5.18 (2H, s), 6.79 (1H, s), 7.23 (2H, br s), 7.39 (5H, s), 9.45 (1H, d, J=8Hz).

Example 13

Potassium (3S, 4R)-(−)-3-[(Z)-2-(2-amino-4-thiazolyl)-2-n-propoxyiminoacetamide]-4-fluoromethyl-2-oxo-1-azetidinesulfonate:—

Substantially in accordance with the method of Example 10, 350 mg (78.2%) of the title compound was obtained by using (Z) - 2 - (2 - amino - 4 - thiazolyl) - 2 - n - propoxyiminoacetic acid.

$[\alpha]_D^{20}$: −23° (c = 1, $H_2O$)

IR (KBr): $cm^{-1}$

3450, 1775, 1665, 1620, 1530, 1270, 1245, 1050.

NMR (DMSO-$d_6$): δ in ppm

0.89 (3H, t, J=7Hz), 1.62 (2H, m), 3.7—4.2 (1H, m), 4.02 (2H, t, J=4Hz), 4.3—5.3 (3H, m), 6.73 (1H, s), 7.2 (2H, br s), 9.31 (1H, d, J=8Hz).

Example 14

Potassium (3S, 4R)-(−)-3-[(Z)-2-(2-amino-4-thiazolyl)-2-(2-propenyloxyimino)acetamide]-4-fluoromethyl-2-oxo-1-azetidinesulfonate:—

Substantially in accordance with the method of Example 10, 290 mg (65.1%) of the title compound was obtained by using (Z) - 2 - (2 - amino - 4 - thiazolyl) - 2 - (2 - propenyloxyimino)acetic acid.

$[\alpha]_D^{20}$: −25.6° (c = 1, $H_2O$)

IR (KBr): $cm^{-1}$

3450, 1770, 1660, 1620, 1270, 1240, 1050, 1005.

NMR (DMSO-$d_6$): δ in ppm

3.7—4.2 (1H, m), 4.3—5.5 (7H, m), 6.76 (1H, s), 7.22 (2H, br s), 9.36 (1H, d, J=8Hz).

Example 15

Potassium (3S, 4R)-(−)-3-[(Z)-2-(2-amino-4-thiazolyl)-2-cyanomethoxyiminoacetamide]-4-fluoromethyl-2-oxo-1-azetidinesulfonate:—

Substantially in accordance with the method of Example 10, 310 mg (69.7%) of the title compound was obtained by using (Z) - 2 - (2 - amino - 4 - thiazolyl) - 2 - cyanomethoxyiminoacetic acid.

$[\alpha]_D^{20}$: −25.6° (c = 1, $H_2O$)

IR (KBr): $cm^{-1}$

3450, 1770, 1660, 1620, 1530, 1270, 1240, 1050, 1020.

NMR (DMSO-$d_6$): δ in ppm

3.7—4.2 (1H, m), 4.3—5.2 (3H, m), 5.06 (2H, s), 6.92 (1H, s), 7.34 (2H, br s), 9.60 (1H, d, J=8Hz).

Example 16

Potassium (3S, 4R)-(−)-3-[(Z)-2-(2-amino-4-thiazolyl)-2-cyclopentyloxyiminoacetamide]-4-fluoromethyl-2-oxo-1-azetidinesulfonate:—

Substantially in accordance with the method of Example 10, 360 mg (76%) of the title compound was obtained by using (Z) - 2 - (2 - amino - 4 - thiazolyl) - 2 - cyclopentyloxyiminoacetic acid.

$[\alpha]_D^{20}$: −15.6° (c = 1, $H_2O$)

IR (KBr): $cm^{-1}$

3450, 1775, 1660, 1620, 1530, 1270, 1240, 1050, 990.

NMR (DMSO-$d_6$): δ in ppm

1.7 (8H, m), 3.7—4.2 (1H, m), 4.3—5.2 (4H, m), 6.73 (1H, s), 7.23 (2H, br s), 9.24 (1H, d, J=8Hz).

Example 17

Potassium (3S, 4R)-(−)-3-[(Z)-2-(2-amino-4-thiazolyl)-2-isopropoxyiminoacetamide]-4-fluoromethyl-2-oxo-1-azetidinesulfonate:—

Substantially in accordance with the method of Example 10, 300 mg (67.1%) of the title compound was obtained by using (Z) - 2 - (2 - amino - 4 - thiazolyl) - 2 - isopropoxyiminoacetic acid.

$[\alpha]_D^{20}$: −22.7° (c = 1, $H_2O$)

IR (KBr): $cm^{-1}$

3450, 1775, 1660, 1620, 1530, 1270, 1240, 1050, 985.

NMR (DMSO-$d_6$): δ in ppm

1.20 (6H, d, J=6Hz), 3.7—4.2 (1H, m), 4.2—5.2 (4H, m), 6.71 (1H, s), 7.20 (2H, br s), 9.22 (1H, d, J=8Hz).

Example 18

Potassium (3S, 4R)-(−)-3-[(Z)-2-(2-amino-4-thiazolyl)-2-(2-propynyloxyimino)acetamide]-4-fluoromethyl-2-oxo-1-azetidinesulfonate:—

Substantially in accordance with the method of Example 10, 310 mg (69.9%) of the title compound was obtained by using (Z) - 2 - (2 - amino - 4 - thiazolyl) - 2 - (2 - propynyloxyimino)acetic acid.

$[\alpha]_D^{20}$: −28.2° (c = 1, $H_2O$)

IR (KBr): $cm^{-1}$

3450, 2120, 1770, 1660, 1620, 1530, 1265, 1240; 1050, 1015, 1005.

NMR (DMSO-$d_6$): δ in ppm

3.48 (1H, t, J=2Hz), 3.7—4.2 (1H, m), 4.3—5.2 (5H, m), 6.80 (1H, s), 7.23 (2H, br s), 9.41 (1H, d, J=8Hz).

Example 19

Potassium (3S, 4R)-(−)-3-[(Z)-2-(2-amino-4-thiazolyl)-2-carboxymethoxyiminoacetamide]-4-fluoromethyl-2-oxo-1-acetidinesulfonate:—

574 mg (1 mmole) of tetra-n-butylammonium (3S, 4R) - (−) - 3 - Cbz - amino - 4 - fluoromethyl - 2 - oxo - 1 - azetidinesulfonate was dissolved in 20 ml of DMF, and 170 mg of 10% palladium carbon was added. The tetra-n-butyl ammonium salt was thus hydrogenated at room temperature for 2 hours. The catalyst was separated by filtration. To the filtrate were added 411 mg (1 mmole) of (Z) - 2 - (2 - amino - 4 - thiazolyl) - 2 - diphenylmethoxycarbonylmethoxyiminoacetic acid, 148 mg (1.1 mmoles) of 1-hydroxybenzotriazole (HOBt) and 206 mg (1 mmole) of N,N-dicyclohexylcarbodiimide and the mixture was stirred at room temperature for 18 hours. The reaction mixture was evaporated under reduced pressure, and methylene chloride was added to the residue. The insoluble material was removed by filtration. The filtrate was chromatographed on a silica gel flash column using acetone-methylene chloride (3:7 to 6:4) as an eluent. Fractions containing the desired product were concentrated to dryness. To the residue was added 2.9 ml of anisole, and the mixture was cooled to −15°C. Cold trifluoroacetic acid (14.4 ml) was added, and the mixture was stirred at less than 0°C for 15 minutes. The trifluoroacetic acid was evaporated off under reduced pressure. Ethyl acetate was added to the residue, and the precipitate was collected by filtration. The precipitate was suspended in 20 ml of water, and the pH of the suspension was adjusted to 5.0 with 0.4N KOH with ice cooling and stirring. The solution was lyophilized to give 430 mg (92.3%) of the title compound as a white powder.

$[\alpha]_D^{20}$: −19° (c = 1, $H_2O$)

IR (KBr): $cm^{-1}$

3450, 1770, 1660, 1610, 1535, 1270, 1245, 1050.

NMR ($DMSO-d_6$): δ in ppm

3.8—4.3 (1H, m), 4.33 (2H, s), 4.4—5.3 (3H, m), 6.85 (1H, s), 7.20 (2H, br s), 11.6 (1H, d, J=8Hz).

### Example 20

N-Cbz-L-γ-fluorothreonineamide:—

(A) A 50% dimethylformamide-water solution of 13.7 g (0.1 mole) of L - (−) - γ - fluorothreonine and 20.9 ml (0.15 mole) of triethylamine was cooled, and while maintaining it at 5 to 10°C, 21.6 ml (0.15 mole) of benzyl chloroformate was added dropwise with stirring. The mixture was stirred at this temperature for 1 hour. The reaction mixture was then poured into 350 ml of ice water, and washed with 200 ml of ethyl acetate. The aqueous layer was adjusted to pH 2.5 with 6N hydrochloric acid and extracted with ethyl acetate to give 25.7 g (95%) of N - Cbz - L - γ - fluorothreonine.

(B) Under ice cooling, 21.6 g (0.105 mole) of N,N'-dicyclohexylcarbodiimide (DCC) was added to a solution of 27.1 g (0.1 mole) of N - Cbz - L - γ - fluorothreonine and 16.1 g (0.11 mole) of N-hydroxysuccinimide (HOSu) in 200 ml of tetrahydrofuran (THF). The mixture was stirred at room temperature for 2 hours. The precipitated N,N-dicyclohexylurea was separated by filtration. The filtrate was added dropwise with stirring to an ice-cooled solution consisting of 32 ml of 7.5N aqueous ammonia and 32 ml of THF, and the mixture was stirred for 2 hours. The reaction mixture was evaporated under reduced pressure. The residue was dissolved in ethyl acetate and washed with a saturated aqueous sodium chloride solution containing 5% sodium hydrogen carbonate. Ethyl acetate was evaporated under reduced pressure, and the precipitated crystals were washed with a small amount of ethyl acetate to give 23.2 g (90%) of the title compound.

Mp: 141—145°C

$[\alpha]_D^{20}$: +9.4° (c = 1, $C_2H_5OH$)

IR (KBr): $cm^{-1}$

3420, 3310, 3220, 1690, 1640, 1535, 1420, 1300, 1250, 1060, 1015, 870, 695.

NMR ($DMSO-d_6$): δ in ppm

4.0—4.30 (3H, m), 4.60 (1H, m), 5.08 (2H, s), 5.40 (1H, d, J=5Hz), 6.88 (1H, d, J=8Hz), 7.20 (1H, br s), 7.30—7.50 (6H, s).

### Example 21

N-Cbz-O-mesyl-L-γ-fluorothreonineamide:—

3.36 g (13 millimoles) of N - Cbz - L - γ - fluorothreonineamide was dissolved in 13 ml of anhydrous pyridine. While the solution was stirred at less than 5°C, 1.2 ml (15.3 moles) of methanesulfonyl chloride was added dropwise. The mixture was continuously stirred at this temperature for 2 hours. The reaction mixture was poured into 20 ml of ice water and stirred for 30 minutes. The precipitated crystals were collected by filtration to give 3.79 g (83.7%) of the title compound.

IR (KBr): $cm^{-1}$

3430, 3320, 1670, 1620, 1535, 1350, 1250, 1185, 1070, 1050, 970, 930, 820, 750, 695.

NMR ($DMSO-d_6$): δ in ppm

3.16 (3H, s), 4.60 (1H, dd, J=9 & 5Hz), 4.70 (2H, dd, J=5 & 3.7Hz), 4.9—5.2 (3H, m), 7.35—7.50 (6H, m), 7.55—7.80 (2H, m).

### Example 22

N-Cbz-O-mesyl-L-γ-fluorothreonine-(N-sulfo)-amidetetra-n-butylammonium salt:—

Chlorosulfonic acid (2.99 ml) was added to a solution of 8.84 ml of 2-picoline in 45 ml of methylene chloride with stirring while maintaining the solution at less than 5°C under ice cooling. A suspension of 3.79 g of N - Cbz - O - mesyl - L - γ - fluorothreonineamide in 60 ml of methylene chloride was added to the resulting solution, and the mixture was boiled under reflux for 16 hours. The reaction mixture was cooled, and poured into 300 ml of a 0.5 M sodium phosphate, monobasic solution (pH 4.5). The aqueous layer was separated, and 5.08 g of tetra-n-butylammonium hydrogen sulfate was added to it. The resulting

solution was extracted with two 74 ml portions of methylene chloride. The extracts were evaporated to dryness under reduced pressure to give 5.65 g (74%) of the title compound as a foam.

NMR (DMSO-$d_6$): δ in ppm

0.94 (12H, t, J=6.2Hz), 1.1—1.8 (16H, m), 3.05—3.50 (11H, m), 4.50 (1H, m), 4.52 (2H, dd, J=47.5 & 3.7Hz), 5.05—5.30 (3H, m), 7.30—7.50 (6H, s), 9.96 (1H, br s).

## Example 23

Tetra-n-butylammonium (3S, 4R)-(−)-3-Cbz-amino-4-fluoromethyl-2-oxo-1-azetidinesulfonate:—

A solution of 3.75 g (5.6 mmoles) of N - Cbz - O - mesyl - L - γ - fluorothreonine (N-sulfo)amide tetra-n-butylammonium salt in 9 ml of 1,2-dichloroethane was added to a mixture, being boiled under reflux, of 2.0 g of potassium carbonate, 7.2 ml of water and 58 ml of 1,2-dichloroethane, and boiled under reflux for 20 minutes. The reaction mixture was cooled, and methylene chloride was added to it. The organic layer was separated, and evaporated under reduced pressure to give an oily residue. The residue was chromatographed on a silica gel flash column using ethyl acetate-acetone (4:1) as an eluent. Fractions containing the desired product were collected and concentrated under reduced pressure. The crystalline residue was taken and washed with a small amount of ethyl acetate to obtain 0.51 g (15.9%) of the title compound.

## Example 24

Potassium (3S, 4R)-(−)-3-[(Z)-2-(1-carboxy-1-cyclobutoxyimino)acetamide]-4-fluoromethyl-2-oxo-1-azetidinesulfonate:—

A solution of 631 mg (1.1 mmoles) of tetra-n-butylammonium (3S, 4R) - (−) - 3 - Cbz - amino - 4 - fluoromethyl - 2 - oxoazetidine - 1 - sulfonate in 20 ml of dimethylformamide (DMF) was subjected to catalytic reduction in the presence of 190 mg of 10% palladium carbon to give a DMF solution of (3S, 4R) - (−) - 3 - amino - 4 - fluoromethyl - 2 - oxoazetidine - 1 - sulfonic acid. To the solution were added 497 mg (1.1 mmoles) of 2 - (2 - amino - 4 - thiazolyl) - 2 - (1 - diphenylmethoxycarbonyl - 1 - cyclobutoxyimino)acetic acid, 164 mg (1.2 mmoles) of 1-hydroxybenzotriazole (HOBt) and 227 mg (1.1 mmoles) of N,N'-dicyclohexylcarbodiimide (DCC), and the mixture was stirred at room temperature for 20 hours. The reaction mixture was evaporated to dryness under reduced pressure. Methylene chloride was added to the residue, and the insoluble material was removed by filtration. The solution was chromatographed on a silica gel flash column using acetone-methylene chloride (3:7—4.5:5.5) as an eluent. To the resulting solid, 2.5 ml of anisole was added. The mixture was cooled to −15°C, and 12.6 ml of trifluoroacetic acid was added. The mixture was stirred at 0°C for 15 minutes. Ethyl acetate (50 ml) and 10 ml of methanol were added to the reaction mixture, and the mixture was concentrated to about 5 ml under reduced pressure. Ethyl acetate (30 ml) was added to the concentrate, and the precipitated insoluble material was collected by filtration. The insoluble material was suspended in 20 ml of water, and dissolved by adjusting its pH to 6 with 0.5N potassium hydroxide. The solution was lyophilized, dissolved in a small amount of water, and passed through a column packed with 25 ml of Diaion HP—20. The column was eluted with water. Fractions containing the desired product were lyophilized to give 180 mg (yield 33.2%) of the title compound.

$[\alpha]_D^{20}$: −15.0° (c = 1, $H_2O$)

IR (KBr): $cm^{-1}$

1770, 1660, 1585, 1535, 1395, 1270, 1245, 1205, 1170, 1120, 1050.

NMR (DMSO-$d_6$): δ in ppm

1.6—2.5 (6H, m), 3.8—4.2 (1H, m), 4.3—5.2 (3H, m), 6.80 (1H, s), 7.22 (2H, br s), 11.67 (1H, d, J=8Hz).

## Example 25

Potassium (3S, 4R)-(−)-3-[(Z)-2-(2-amino-4-thiazolyl)-2-(1-carboxyl-1-cyclopentoxyimino)acetamide]-4-fluoromethyl-2-oxo-1-azetidinesulfonate:—

Substantially in accordance with the method of Example 24, 170 mg (yield 30.6%) of the title compound was obtained from 1.1 mmoles of (3S, 4R) - (−) - 3 - amino - 4 - fluoromethyl - 2 - oxo - 1 - azetidinesulfonic acid and 512 mg (1.1 mmoles) of (Z) - 2 - (2 - amino - 4 - thiazolyl) - 2 - (1 - diphenyl methoxycarbonyl - 1 - cyclopentoxyimino)acetic acid.

$[\alpha]_D^{20}$: −10.4° (c = 1, $H_2O$)

IR (KBr): $cm^{-1}$

1775, 1660, 1585, 1535, 1395, 1270, 1245, 1200, 1050.

NMR (DMSO-$d_6$): δ in ppm

1.4—2.3 (8H, m), 3.8—4.2 (1H, m), 4.3—5.2 (3H, m), 6.80 (1H, s), 7.20 (2H, br s), 12.1 (1H, d, J=8Hz).

**Claims for the Contracting States: BE CH DE FR GB IT LI LU NL SE**

1. An optically active compound represented by the following formula

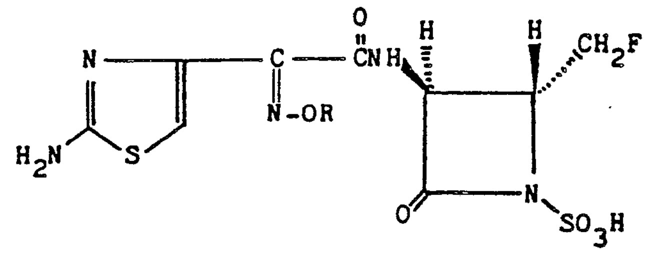

...(1)

wherein R represents a group selected from the class consisting of linear or branched $C_1$—$C_4$ alkyl groups, $C_3$—$C_4$ alkenyl groups, $C_3$—$C_4$ alkynyl groups, a cyano-substituted methyl group, $C_3$—$C_6$ cycloalkyl groups (the latter being optionally substituted by carboxy), carboxy-substituted $C_1$—$C_5$ alkyl groups, a benzyl group and a phenyl group,
and a pharmaceutically acceptable salt thereof.

2. The compound and its salt according to claim 1 wherein the compound of formula (1) is (3S, 4R) - (−) - 3 - [(Z) - 2 - (2 - amino - 4 - thiazolyl) - 2 - methoxyiminoacetamide] - 4 - fluoromethyl - 2 - oxo - 1 - azetidinesulfonic acid.

3. The compound and its salt according to claim 1 wherein the compound of formula (1) is (3S, 4R) - (−) - 3 - [(Z) - 2 - (2 - amino - 4 - thiazolyl) - 2 - ethoxyiminoacetamide] - 4 - fluoromethyl - 2 - oxo - 1 - azetidinesulfonic acid.

4. The compound and its salt according to claim 1 wherein the compound of formula (1) is (3S, 4R) - (−) - 3 - [(Z) - 2 - (2 - amino - 4 - thiazolyl) - 2 - n - propoxyiminoacetamide] - 4 - fluoromethyl - 2 - oxo - 1 - azetidinesulfonic acid.

5. The compound and its salt according to claim 1 wherein the compound of formula (1) is (3S, 4R) - (−) - 3 - [(Z) - 2 - (2 - amino - 4 - thiazolyl) - 2 - isopropoxyiminoacetamide] - 4 - fluoromethyl - 2 - oxo - 1 - azetidinesulfonic acid.

6. The compound and its salt according to claim 1 wherein the compound of formula (1) is (3S, 4R) - (−) - 3 - [(Z) - 2 - (2 - amino - 4 - thiazolyl) - 2 - (2 - propenyloxyimino)acetamide] - 4 - fluoromethyl - 2 - oxo - 1 - azetidinesulfonic acid.

7. The compound and its salt according to claim 1 wherein the compound of formula (1) is (3S, 4R) - (−) - 3 - [(Z) - 2 - (2 - amino - 4 - thiazolyl) - 2 - (2 - propynyloxyimino)acetamide] - 4 - fluoromethyl - 2 - oxo - 1 - azetidinesulfonic acid.

8. The compound and its salt according to claim 1 wherein the compound of formula (1) is (3S, 4R) - (−) - 3 - [(Z) - 2 - (2 - amino - 4 - thiazolyl) - 2 - carboxymethoxyiminoacetamide] - 4 - fluoromethyl - 2 - oxo - 1. - azetidinesulfonic acid.

9. The compound and its salt according to claim 1 wherein the compound of formula (1) is (3S, 4R) - (−) - 3 - [(Z) - 2 - (2 - amino - 4 - thiazolyl) - 2 - cyanomethoxyiminoacetamide] - 4 - fluoromethyl - 2 - oxo - 1 - azetidinesulfonic acid.

10. The compound and its salt according to claim 1 wherein the compound of formula (1) is (3S, 4R) - (−) - 3 - [(Z) - 2 - (2 - amino - 4 - thiazolyl) - 2 - (1 - carboxy - 1 - methylethoxyimino)acetamide] - 4 - fluoromethyl - 2 - oxo - 1 - azetidinesulfonic acid.

11. The compound and its salt according to claim 1 wherein the compound of formula (1) is (3S, 4R) - (−) - 3 - [(Z) - 2 - (2 - amino - 4 - thiazolyl) - 2 - cyclopentyloxyiminoacetamide] - 4 - fluoromethyl - 2 - oxo - 1 - azetidinesulfonic acid.

12. The compound and its salt according to claim 1 wherein the compound of formula (1) is (3S, 4R) - (−) - 3 - [(Z) - 2 - (2 - amino - 4 - thiazolyl) - 2 - benzyloxyiminoacetamide] - 4 - fluoromethyl - 2 - oxo - 1 - azetidinesulfonic acid.

13. The compound and its salt according to claim 1 wherein the compound of formula (1) is (3S, 4R) - (−) - 3 - [(Z) - 2 - (2 - amino - 4 - thiazolyl) - 2 - phenoxyiminoacetamide] - 4 - fluoromethyl - 2 - oxo - 1 - azetidinesulfonic acid.

14. The compound and its salt according to claim 1 wherein the compound of formula (1) is (3S, 4R) - (−) - 3 - [(Z) - 2 - (2 - amino - 4 - thiazolyl) - 2 - (1 - carboxy - 1 - methylpropoxyimino)acetamide] - 4 - fluoromethyl - 2 - oxo - 1 - azetidinesulfonic acid.

15. The compound and its salt according to claim 1 wherein the compound of formula (1) is (3S, 4R) - (−) - 3 - [(Z) - 2 - (2 - amino - 4 - thiazolyl) - 2 - (1 - carboxy - 1 - cyclopropoxyimino)acetamide] - 4 - fluoromethyl - 2 - oxo - 1 - azetidinesulfonic acid.

16. The compound and its salt according to claim 1 wherein the compound of formula (1) is (3S, 4R) - (−) - 3 - [(Z) - 2 - (2 - amino - 4 - thiazolyl) - 2 - (1 - carboxy - 1 - cyclobutoxyimino)acetamide] - 4 - fluoromethyl - 2 - oxo - 1 - azetidinesulfonic acid.

17. The compound and its salt according to claim 1 wherein the compound of formula (1) is (3S, 4R) -

(−) - 3 - [(Z) - 2 - (2 - amino - 4 - thiazolyl) - 2 - (1 - carboxy - 1 - cyclopentoxyimino)acetamide] - 4 - fluoromethyl - 2 - oxo - 1 - azetidinesulfonic acid.

18. The compound and its salt according to claim 1 wherein the compound of formula (1) is (3S, 4R) - (−) - 3 - [(Z) - 2 - (2 - amino - 4 - thiazolyl) - 2 - (1 - carboxy - 1 - cyclohexyloxyimino)acetamide] - 4 - fluoromethyl - 2 - oxo - 1 - azetidinesulfonic acid.

19. An antibacterial composition composed of an antibacterially effective amount of a compound of claim 1 and a pharmaceutically acceptable diluent or carrier.

20. A process for producing a compound of claim 1 which comprises reacting a compound represented by the following formula

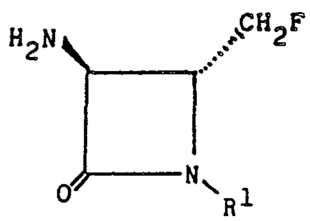

...(A)

wherein $R^1$ represents a hydrogen atom, $—SO_3H$ or an inorganic salt or organic amine salt of $—SO_3H$, with a reactive derivative resulting from the activation of the carboxy group of a compound of the following formula

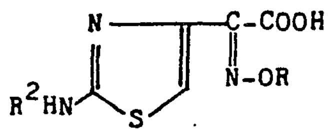

...(B)

wherein $R^2$ represents a hydrogen atom or a protective group for the amino group and R is as defined in claim 1 provided that when R has a carboxy substituent it is protected by a protective group for the carboxy group;
when $R^1$ in the compound of formula (A) is a hydrogen atom, sulfonating the reaction product; and then eliminating the protective groups.

**Claims for the Contracting State: AT**

1. A process for producing an optically active compound represented by the following formula

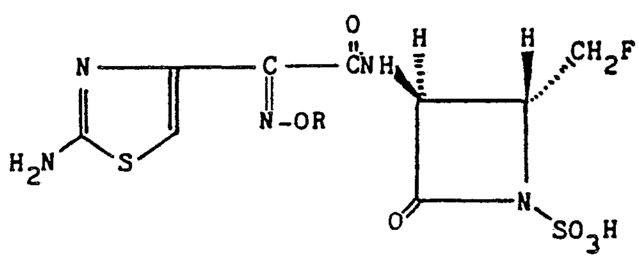

...(1)

wherein R represents a group selected from the class consisting of linear or branched $C_1—C_4$ alkyl groups, $C_3—C_4$ alkenyl groups, $C_3—C_4$ alkynyl groups, a cyano-substituted methyl group, $C_3—C_6$ cycloalkyl groups (the latter being optionally substituted by carboxy), carboxy-substituted $C_1—C_5$ alkyl groups, a benzyl group and a phenyl group,
and its pharmaceutically acceptable salt thereof, which comprises reacting a compound represented by the following formula

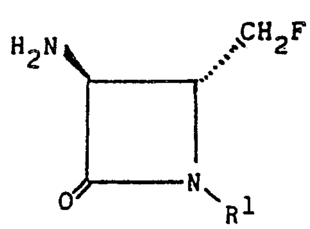

...(A)

wherein $R^1$ represents a hydrogen atom, $—SO_3H$ or an inorganic salt or organic amine salt of $—SO_3H$,

with a reactive derivative resulting from the activation of the carboxy group of a compound of the following formula

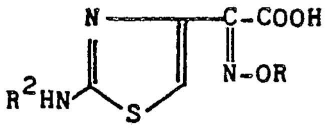

...(B)

wherein $R^2$ represents a hydrogen atom or a protective group for the amino group and R is as defined with regard to formula (1), provided that when R has a carboxy substituent it is protected by a protective group for the carboxy group;
when $R^1$ in the compound of formula (A) is a hydrogen atom, sulfonating the reaction product; and then eliminating the protective groups.

2. The process according to claim 1 wherein the compound of formula (1) is (3S, 4R) - (−) - 3 - [(Z) - 2 - (2 - amino - 4 - thiazolyl) - 2 - methoxyiminoacetamide] - 4 - fluoromethyl - 2 - oxo - 1 - azetidinesulfonic acid.

3. The process according to claim 1 wherein the compound of formula (1) is (3S, 4R) - (−) - 3 - [(Z) - 2 - (2 - amino - 4 - thiazolyl) - 2 - ethoxyiminoacetamide] - 4 - fluoromethyl - 2 - oxo - 1 - azetidinesulfonic acid.

4. The process according to claim 1 wherein the compound of formula (1) is (3S, 4R) - (−) - 3 - [(Z) - 2 - (2 - amino - 4 - thiazolyl) - 2 - n - propoxyiminoacetamide] - 4 - fluoromethyl - 2 - oxo - 1 - azetidinesulfonic acid.

5. The process according to claim 1 wherein the compound of formula (1) is (3S, 4R) - (−) - 3 - [(Z) - 2 - (2 - amino - 4 - thiazolyl) - 2 - isopropoxyiminoacetamide] - 4 - fluoromethyl - 2 - oxo - 1 - azetidinesulfonic acid.

6. The process according to claim 1 wherein the compound of formula (1) is (3S, 4R) - (−) - 3 - [(Z) - 2 - (2 - amino - 4 - thiazolyl) - 2 - (2 - propenyloxyimino)acetamide] - 4 - fluoromethyl - 2 - oxo - 1 - azetidinesulfonic acid.

7. The process according to claim 1 wherein the compound of formula (1) is (3S, 4R) - (−) - 3 - [(Z) - 2 - (2 - amino - 4 - thiazolyl) - 2 - (2 - propynyloxyimino)acetamide] - 4 - fluoromethyl - 2 - oxo - 1 - azetidinesulfonic acid.

8. The process according to claim 1 wherein the compound of formula (1) is (3S, 4R) - (−) - 3 - [(Z) - 2 - (2 - amino - 4 - thiazolyl) - 2 - carboxymethoxyiminoacetamide] - 4 - fluoromethyl - 2 - oxo - 1 - azetidinesulfonic acid.

9. The process according to claim 1 wherein the compound of formula (1) is (3S, 4R) - (−) - 3 - [(Z) - 2 - (2 - amino - 4 - thiazolyl) - 2 - cyanomethoxyiminoacetamide] - 4 - fluoromethyl - 2 - oxo - 1 - azetidinesulfonic acid.

10. The process according to claim 1 wherein the compound of formula (1) is (3S, 4R) - (−) - 3 - [(Z) - 2 - (2 - amino - 4 - thiazolyl) - 2 - (1 - carboxy - 1 - methylethoxyimino)acetamide] - 4 - fluoromethyl - 2 - oxo - 1 - azetidinesulfonic acid.

11. The process according to claim 1 wherein the compound of formula (1) is (3S, 4R) - (−) - 3 - [(Z) - 2 - (2 - amino - 4 - thiazolyl) - 2 - cyclopentyloxyiminoacetamide] - 4 - fluoromethyl - 2 - oxo - 1 - azetidinesulfonic acid.

12. The process according to claim 1 wherein the compound of formula (1) is (3S, 4R) - (−) - 3 - [(Z) - 2 - (2 - amino - 4 - thiazolyl) - 2 - benzyloxyiminoacetamide] - 4 - fluoromethyl - 2 - oxo - 1 - azetidinesulfonic acid.

13. The process according to claim 1 wherein the compound of formula (1) is (3S, 4R) - (−) - 3 - [(Z) - 2 - (2 - amino - 4 - thiazolyl) - 2 - phenoxyiminoacetamide] - 4 - fluoromethyl - 2 - oxo - 1 - azetidinesulfonic acid.

14. The process according to claim 1 wherein the compound of formula (1) is (3S, 4R) - (−) - 3 - [(Z) - 2 - (2 - amino - 4 - thiazolyl) - 2 - (1 - carboxy - 1 - methylpropoxyimino)acetamide] - 4 - fluoromethyl - 2 - oxo - 1 - azetidinesulfonic acid.

15. The process according to claim 1 wherein the compound of formula (1) is (3S, 4R) - (−) - 3 - [(Z) - 2 - (2 - amino - 4 - thiazolyl) - 2 - (1 - carboxy - 1 - cyclopropoxyimino)acetamide] - 4 - fluoromethyl - 2 - oxo - 1 - azetidinesulfonic acid.

16. The process according to claim 1 wherein the compound of formula (1) is (3S, 4R) - (−) - 3 - [(Z) - 2 - (2 - amino - 4 - thiazolyl) - 2 - (1 - carboxy - 1 - cyclobutoxyimino)acetamide] - 4 - fluoromethyl - 2 - oxo - 1 - azetidinesulfonic acid.

17. The process according to claim 1 wherein the compound of formula (1) is (3S, 4R) - (−) - 3 - [(Z) - 2 - (2 - amino - 4 - thiazolyl) - 2 - (1 - carboxy - 1 - cyclopentoxyimino)acetamide] - 4 - fluoromethyl - 2 - oxo - 1 - azetidinesulfonic acid.

18. The process according to claim 1 wherein the compound of formula (1) is (3S, 4R) - (−) - 3 - [(Z) - 2 - (2 - amino - 4 - thiazolyl) - 2 - (1 - carboxy - 1 - cyclohexyloxyimino)acetamide] - 4 - fluoromethyl - 2 - oxo - 1 - azetidinesulfonic acid.

**Patentansprüche für die Vertragsstaaten: BE CH DE FR GB IT LI LU NL SE**

1. Optisch aktive Verbindung der folgenden Formel

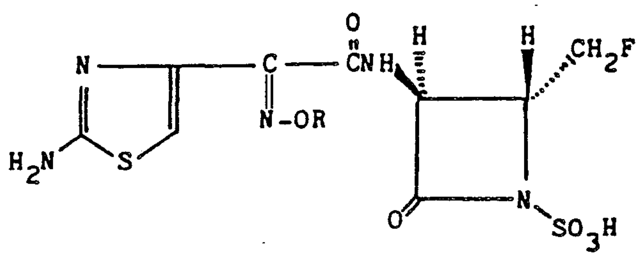

...(1)

worin R eine Gruppe bedeutet, die ausgewählt ist unter linearen oder verzweigten $C_{1-4}$-Alkylgruppen, $C_{3-4}$-Alkenylgruppen, $C_{3-4}$-Alkinylgruppen, einer cyano substituierten Methylgruppe, $C_{3-6}$-Cycloalkylgruppen (wobei die letztere gegebenenfalls durch Carboxy substituiert sein kann), carboxy substituierten $C_{1-5}$-Alkylgruppen, einer Benzylgruppe und einer Phenylgruppe,
und eines ihrer pharmazeutisch annehmbaren Salze.

2. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - methoxyiminoacetamid] - 4 - fluormethyl - 2 - oxo - 1 - azetidinsulfonsäure ist.

3. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - ethoxyiminoacetamid] - 4 - fluormethyl - 2 - oxo - 1 - azetidinsulfonsäure ist.

4. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - n - propoxyiminoacetamid] - 4 - fluormethyl - 2 - oxo - 1 - azetidinsulfonsäure ist.

5. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - isopropoxyiminoacetamid] - 4 - fluormethyl - 2 - oxo - 1 - azetidinsulfonsäure ist.

6. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (2-propenyloxyimino)-acetamid] - 4 - fluormethyl - 2 - oxo - 1 - azetidinsulfonsäure ist.

7. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (2-propinyloxyimino) - acetamid] - 4 - fluormethyl - 2 - oxo - 1 - azetidinsulfonsäure ist.

8. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - carboxymethoxyiminoacetamid] - 4 - fluormethyl - 2 - oxo - 1 - azetidinsulfonsäure ist.

9. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - cyanomethoxyiminoacetamid] - 4 - fluormethyl - 2 - oxo - 1 - azetidinsulfonsäure ist.

10. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - methylethoxyimino) - acetamid] - 4 - fluormethyl - 2 - oxo - 1 - azetidinsulfonsäure ist.

11. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - cyclopentyloxyiminoacetamid] - 4 - fluormethyl - 2 - oxo - 1 - azetidinsulfonsäure ist.

12. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - benzyloxyiminoacetamid] - 4 - fluormethyl - 2 - oxo - 1 - azetidinsulfonsäure ist.

13. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - phenoxyiminoacetamid] - 4 - fluormethyl - 2 - oxo - 1 - azetidinsulfonsäure ist.

14. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - methylpropoxyimino) - acetamid] - 4 - fluormethyl - 2 - oxo - 1 - azetidinsulfonsäure ist.

15. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclopropoxyimino) - acetamid] - 4 - fluormethyl - 2 - oxo - 1 - azetidinsulfonsäure ist.

16. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclobutoxyimino) - acetamid] - 4 - fluormethyl - 2 - oxo - 1 - azetidinsulfonsäure ist.

17. Verbindung oder ihr Salz nach Anspruch 1, dadurch gekennzeichnet, daß die Verbindung der

Formel (1) (3S, 4R) - (−) - 3 - [(Z) - 2 - (2 - Amino - 4 - thiazolyl) - 2 - (1 - carboxy - 1 - cyclopentoxyimino) - acetamid] - 4 - fluormethyl - 2 - oxo - 1 - azetidinsulfonsäure ist.

18. Verbindung oder ihr Salz nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclohexyloxyimino) - acetamid] - 4 - fluormethyl - 2 - oxo - 1 - azetidinsulfonsäure ist.

19. Antibakterielle Zubereitung, dadurch gekennzeichnet, daß sie eine antibakteriell wirksame Menge einer Verbindung nach Anspruch 1 und ein pharmazeutisch annehmbares Verdünnungsmittel oder Träger enthält.

20. Verfahren zur Herstellung einer Verbindung nach Anspruch 1, dadurch gekennzeichnet, daß man eine Verbindung der folgenden Formel

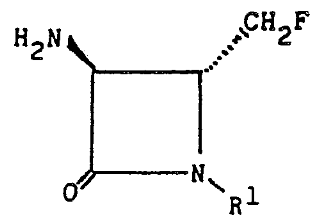

...(A)

worin $R^1$ ein Wasserstoffatom, $—SO_3H$ oder ein anorganisches Salz oder ein organisches Aminsalz von $—SO_3H$ bedeutet,
mit einem reaktiven Derivat umsetzt, das durch Aktivierung der Carboxygruppe einer Verbindung der folgenden Formel

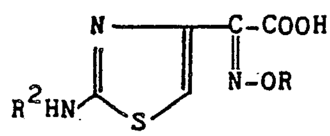

...(B)

erhalten wird, worin $R^2$ ein Wasserstoffatom oder eine Schutzgruppe für die Aminogruppe bedeutet und R die in Anspruch 1 gegebene Definition besitzt, mit der Maßgabe, daß, wenn R einen Carboxy-Substituenten aufweist, dieser durch eine Schutzgruppe für die Carboxygruppe geschützt ist;
und, wenn $R^1$ in der Verbindung der Formel (A) ein Wasserstoffatom bedeutet, das Reaktionsprodukt sulfoniert und dann die Schutzgruppen entfernt.

**Patentansprüche für den Vertragsstaat: AT**

1. Verfahren zur Herstellung einer optisch aktiven Verbindung der allgemeinen Formel

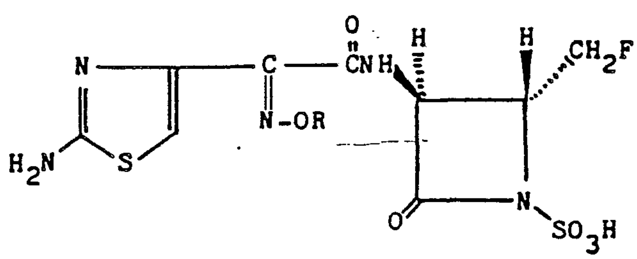

...(1)

worin R eine Gruppe bedeutet, ausgewählt unter linearen oder verzweigten $C_{1-4}$-Alkylgruppen, $C_{3-4}$-Alkenylgruppen, $C_{3-4}$-Alkinylgruppen, einer cyano substituierten Methylgruppe, $C_{3-6}$-Cycloalkylgruppen (wobei die letzteren gegebenenfalls durch Carboxy substituiert sein können), carboxy substituierten $C_{1-5}$-Alkylgruppen, einer Benzylgruppe und einer Phenylgruppe,
und eines seiner pharmazeutisch annehmbaren Salze, dadurch gekennzeichnet, daß man eine Verbindung der folgenden Formel

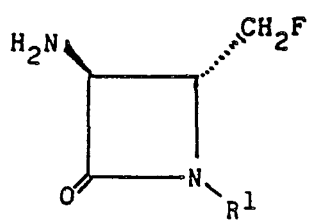

...(A)

worin $R^1$ ein Wasserstoffatom, $—SO_3H$ oder ein anorganisches Salz oder organisches Aminsalz von $—SO_3H$ bedeutet,

mit einem reaktiven Derivat umsetzt, das durch Aktivierung der Carboxygruppe einer Verbindung der folgenden Formel

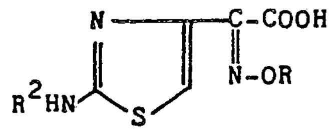

...(B)

erhalten wird, worin $R^2$ ein Wasserstoffatom oder eine Schutzgruppe für die Aminogruppe bedeutet und R die bei Formel (1) gegebene Bedeutung besitzt, mit der Maßgabe, daß, wenn R einen Carboxy-Substituenten aufweist, dieser durch eine Schutzgruppe für die Carboxygruppe geschützt ist;
und, wenn $R^1$ in der Formel (A) ein Wasserstoffatom bedeutet, das Reaktionsprodukt sulfoniert und dann die Schutzgruppen entfernt.

2. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - methoxyiminoacetamid] - 4 - fluormethyl - 2 - oxo - 1 - azetidinsulfonsäure ist.

3. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - ethoxyiminoacetamid] - 4 - fluormethyl - 2 - oxo - 1 - azetidinsulfonsäure ist.

4. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - n - propoxyiminoacetamid] - 4 - fluormethyl - 2 - oxo - 1 - azetidinsulfonsäure ist.

5. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - isopropoxyiminoacetamid] - 4 - fluormethyl - 2 - oxo - 1 - azetidinsulfonsäure ist.

6. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (2-propenyloxyimino)-acetamid] - 4 - fluormethyl - 2 - oxo - 1 - azetidinsulfonsäure ist.

7. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (2-propinyloxyimino) - acetamid] - 4 - fluormethyl - 2 - oxo - 1 - azetidinsulfonsäure ist.

8. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - carboxymethoxyiminoacetamid] - 4 - fluormethyl - 2 - oxo - 1 - azetidinsulfonsäure ist.

9. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - cyanomethoxyiminoacetamid] - 4 - fluormethyl - 2 - oxo - 1 - azetidinsulfonsäure ist.

10. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - methylethoxyimino) - acetamid] - 4 - fluormethyl - 2 - oxo - 1 - azetidinsulfonsäure ist.

11. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - cyclopentyloxyiminoacetamid] - 4 - fluormethyl - 2 - oxo - 1 - azetidinsulfonsäure ist.

12. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - benzyloxyiminoacetamid] - 4 - fluormethyl - 2 - oxo - 1 - azetidinsulfonsäure ist.

13. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - phenoxyiminoacetamid] - 4 - fluormethyl - 2 - oxo - 1 - azetidinsulfonsäure ist.

14. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - methylpropoxyimino) - acetamid] - 4 - fluormethyl - 2 - oxo - 1 - azetidinsulfonsäure ist.

15. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclopropoxyimino) - acetamid] - 4 - fluormethyl - 2 - oxo - 1 - azetidinsulfonsäure ist.

16. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclobutoxyimino) - acetamid] - 4 - fluormethyl - 2 - oxo - 1 - azetidinsulfonsäure ist.

17. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclopentoxyimino) - acetamid] - 4 - fluormethyl - 2 - oxo - 1 - azetidinsulfonsäure ist.

18. Verfahren nach Anspruch 1, dadurch gekennzeichnet, daß die Verbindung der Formel (1) (3S, 4R) - (−) - 3 - [(Z) - 2 - (2 - Amino - 4 - thiazolyl) - 2 - (1 - carboxy - 1 - cyclohexyloxyimino) - acetamid] - 4 - fluormethyl - 2 - oxo - 1 - azetidinsulfonsäure ist.

**Revendications pour les Etats contractants: BE CH DE FR GB IT LI LU NL SE**

1. Composé optiquement actif répondant à la formule suivante

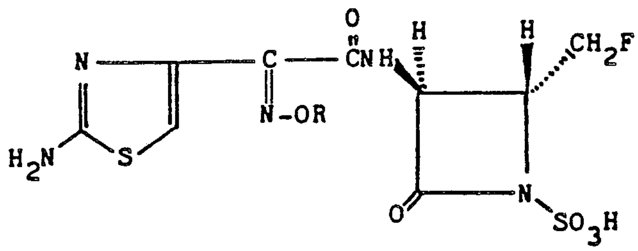

...(1)

dans laquelle R représente un groupe choisi dans la classe constituée des groupes alkyle en $C_1$ à $C_4$, des groupes alcényle en $C_3$ à $C_4$, des groupes alcynyle en $C_3$ à $C_4$ linéaires ou ramifiés, d'un groupe méthyle substitué par un cyano, des groupes cycloalkyle en $C_3$ à $C_6$ (ces derniers étant substitués si on le désire par un carboxy), des groupes alkyle en $C_1$ à $C_5$ substitués par un carboxy, d'un groupe benzyle et d'un groupe phényle,

et, un sel pharmaceutiquement acceptable de celui-ci.

2. Composé et son sel suivant la revendication 1, où le composé répondant à la formule (1) est l'acide (3S, 4R) - (−) - 3 - [(Z) - 2 - (2 - amino - 4 - thiazolyl) - 2 - méthoxyiminoacétamide] - 4 - fluorométhyl - 2 - oxo - 1 - azétidinesulfonique.

3. Composé et son sel suivant la revendication 1, où le composé répondant à la formule (1) est l'acide (3S, 4R) - (−) - 3 - [(Z) - 2 - (2 - amino - 4 - thiazolyl) - 2 - éthoxyiminoacétamide] - 4 - fluorométhyl - 2 - oxo - 1 - azétidinesulfonique.

4. Composé et son sel suivant la revendication 1, où le composé répondant à la formule (1) est l'acide (3S, 4R) - (−) - 3 - [(Z) - 2 - (2 - amino - 4 - thiazolyl) - 2 - n - propoxyiminoacétamide] - 4 - fluorométhyl - 2 - oxo - 1 - azétidinesulfonique.

5. Composé et son sel suivant la revendication 1, où le composé répondant à la formule (1) est l'acide (3S, 4R) - (−) - 3 - [(Z) - 2 - (2 - amino - 4 - thiazolyl) - 2 - isopropoxyiminoacétamide] - 4 - fluorométhyl - 2 - oxo - 1 - azétidinesulfonique.

6. Composé et son sel suivant la revendication 1, où le composé répondant à la formule (1) est l'acide (3S, 4R) - (−) - 3 - [(Z) - 2 - (2 - amino - 4 - thiazolyl) - 2 - (2 - propenyloxyimino)acétamide] - 4 - fluorométhyl - 2 - oxo - 1 - azétidinesulfonique.

7. Composé et son sel suivant la revendication 1, où le composé répondant à la formule (1) est l'acide (3S, 4R) - (−) - 3 - [(Z) - 2 - (2 - amino - 4 - thiazolyl) - 2 - (2 - propynyloxyimino)acétamide]-4 - fluorométhyl - 2 - oxo - 1 - azétidinesulfonique.

8. Composé et son sel suivant la revendication 1, où le composé répondant à la formule (1) est l'acide (3S, 4R) - (−) - 3 - [(Z) - 2 - (2 - amino - 4 - thiazolyl) - 2 - carboxyméthoxyiminoacétamide] - 4 - fluorométhyl - 2 - oxo - 1 - azétidinesulfonique.

9. Composé et son sel suivant la revendication 1, où le composé répondant à la formule (1) est l'acide (3S, 4R) - (−) - 3 - [(Z) - 2 - (2 - amino - 4 - thiazolyl) - 2 - cyanométhoxyiminoacétamide] - 4 - fluorométhyl - 2 - oxo - 1 - azétidinesulfonique.

10. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - méthyléthoxyimino)acétamide] - 4 - fluorométhyl - 2 - oxo - 1 - azétidinesulfonique.

11. Composé et son sel suivant la revendication 1, où le composé répondant à la formule (1) est l'acide (3S, 4R) - (−) - 3 - [(Z) - 2 - (2 - amino - 4 - thiazolyl) - 2 - cyclopentyloxyiminoacétamide]-4 - fluorométhyl - 2 - oxo - 1 - azétidinesulfonique.

12. Composé et son sel suivant la revendication 1, où le composé répondant à la formule (1) est l'acide (3S, 4R) - (−) - 3 - [(Z) - 2 - (2 - amino - 4 - thiazolyl) - 2 - benzyloxyiminoacétamide] - 4 - fluorométhyl - 2 - oxo - 1 - azétidinesulfonique.

13. Composé et son sel suivant la revendication 1, où le composé répondant à la formule (1) est l'acide (3S, 4R) - (−) - 3 - [(Z) - 2 - (2 - amino - 4 - thiazolyl) - 2 - phénoxyiminoacétamide] - 4 - fluorométhyl - 2 - oxo - 1 - azétidinesulfonique.

14. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - méthylpropoxyimino)acétamide] - 4 - fluorométhyl - 2 - oxo - 1 - azétidinesulfonique.

15. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - cyclopropoxyimino)acétamide] - 4 - fluorométhyl - 2 - oxo - 1 - azétidinesulfonique.

16. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - cyclobutoxyimino)acétamide] - 4 - fluorométhyl - 2 - oxo - 1 - azétidinesulfonique.

17. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - cyclopentoxyimino)acétamide] - 4 - fluorométhyl - 2 - oxo - 1 - azétidinesulfonique.

18. Composé et son sel suivant la revendication 1, où le composé répondant à la formule (1) est l'acide (3S, 4R) - (−) - 3 - [(Z) - 2 - (2 - amino - 4 - thiazolyl) - 2 - (1 - carboxy - 1 - cyclohexyloxyimino)acétamide] - 4 - fluorométhyl - 2 - oxo - 1 - azétidinesulfonique.

19. Composition antibactérienne composée d'une quantité efficace contre les bactéries d'un composé suivant la revendication 1, et d'un diluant ou support pharmaceutiquement acceptable.

20. Procédé de préparation d'un composé suivant la revendication 1, qui consiste à faire réagir un composé répondant à la formule suivante

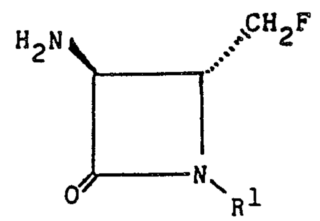

...(A)

dans laquelle $R^1$ représente un atome d'hydrogène, $—SO_3H$ ou un sel minéral ou un sel d'amine organique de $—SO_3H$,
avec un dérivé réactif résultant de l'activation du groupe carboxy d'un composé répondant à la formule suivante

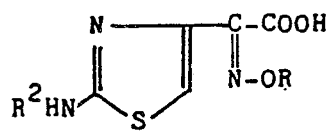

...(B)

dans laquelle $R^2$ représente un atome d'hydrogène, ou un groupe protecteur pour le groupe amino et R est tel que défini dans la revendication 1, sous réserve que lorsque R a un substituant carboxy, il est protégé par un groupe protecteur du groupe carboxy;
lorsque $R^1$ dans le composé répondant à la formule (A) est un atome d'hydrogène, à sulfoner le produit de la réaction; puis à éliminer les groupes protecteurs.

**Revendications pour l'Etat contractant: AT**

1. Procédé de préparation d'un composé optiquement actif répondant à la formule suivante

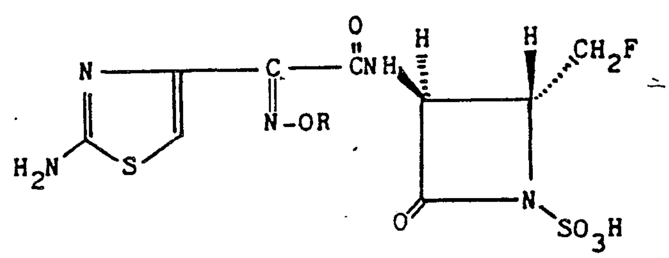

...(1)

dans laquelle R représente un groupe choisi dans la classe constituée des groupes alkyle en $C_1$ à $C_4$, des groupes alcényle en $C_3$ à $C_4$, des groupes alcynyle en $C_3$ à $C_4$ linéaires ou ramifiés, d'un groupe méthyle substitué par un cyano, des groupes cycloalkyle en $C_3$ à $C_6$ (ces derniers étant substitués si on le désire par un carboxy), des groupes alkyle en $C_1$ à $C_5$ substitués par un carboxy, d'un groupe benzyle et d'un groupe phényle,
et son sel pharmaceutiquement acceptable, qui consiste à faire réagir un composé répondant à la formule suivante

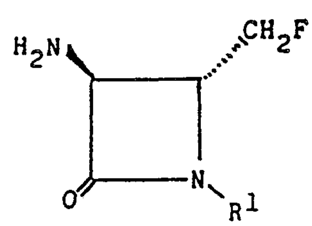

...(A)

dans laquelle $R^1$ représente un atome d'hydrogène, $—SO_3H$ ou un sel minéral ou un sel d'amine organique de $—SO_3H$,

avec un dérivé réactif résultant de l'activation du groupe carboxy d'un composé répondant à la formule suivante

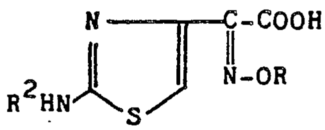

...(B)

dans laquelle $R^2$ représente un atome d'hydrogène ou un groupe protecteur pour le groupe amino et R est tel que défini à propos de la formule (1), sous réserve que lorsque R a un substituant carboxy, il est protégé par un groupe protecteur pour le groupe carboxy;
lorsque $R^1$ dans le composé répondant à la formule (A) est un atome d'hydrogène, à sulfoner le produit de la réaction; puis à éliminer les groupes protecteurs.

2. Procédé suivant la revendication 1, dans lequel le composé répondant à la formule (1) est l'acide (3S, 4R) - (−) - 3 - [(Z) - 2 - (2 - amino - 4 - thiazolyl) - 2 - méthoxyiminoacétamide] - 4 - fluorométhyl - 2 - oxo - 1 - azétidinesulfonique.

3. Procédé suivant la revendication 1, dans lequel le composé répondant à la formule (1) est l'acide (3S, 4R) - (−) - 3 - [(Z) - 2 - (2 - amino - 4 - thiazolyl) - 2 - éthoxyiminoacétamide] - 4 - fluorométhyl - 2 - oxo - 1 - azétidinesulfonique.

4. Procédé suivant la revendication 1, dans lequel le composé répondant à la formule (1) est l'acide (3S, 4R) - (−) - 3 - [(Z) - 2 - (2 - amino - 4 - thiazolyl) - 2 - n - propoxyiminoacétamide] - 4 - fluorométhyl - 2 - oxo - 1 - azétidinesulfonique.

5. Procédé suivant la revendication 1, dans lequel le composé répondant à la formule (1) est l'acide (3S, 4R) - (−) - 3 - [(Z) - 2 - (2 - amino - 4 - thiazolyl) - 2 - isopropoxyiminoacétamide] - 4 - fluorométhyl - 2 - oxo - 1 - azétidinesulfonique.

6. Procédé suivant la revendication 1, dans lequel le composé répondant à la formule (1) est l'acide (3S, 4R) - (−) - 3 - [(Z) - 2 - (2 - amino - 4 - thiazolyl) - 2 - (2 - propenyloxyimino)acétamide] - 4 - fluorométhyl - 2 - oxo - 1 - azétidinesulfonique.

7. Procédé suivant la revendication 1, dans lequel le composé répondant à la formule (1) est l'acide (3S, 4R) - (−) - 3 - [(Z) - 2 - (2 - amino - 4 - thiazolyl) - 2 - (2 - propynyloxyimino)acétamide] - 4 - fluorométhyl - 2 - oxo - 1 - azétidinesulfonique.

8. Procédé suivant la revendication 1, dans lequel le composé répondant à la formule (1) est l'acide (3S, 4R) - (−) - 3 - [(Z) - 2 - (2 - amino - 4 - thiazolyl) - 2 - carboxyméthoxyiminoacétamide] - 4 - fluorométhyl - 2 - oxo - 1 - azétidinesulfonique.

9. Procédé suivant la revendication 1, dans lequel le composé répondant à la formule (1) est l'acide (3S, 4R) - (−) - 3 - [(Z) - 2 - (2 - amino - 4 - thiazolyl) - 2 - cyanométhoxyiminoacétamide] - 4 - fluorométhyl - 2 - oxo - 1 - azétidinesulfonique.

10. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - méthyléthoxyimino)acétamide] - 4 - fluorométhyl - 2 - oxo - 1 - azétidinesulfonique.

11. Procédé suivant la revendication 1, dans lequel le composé répondant à la formule (1) est l'acide (3S, 4R) - (−) - 3 - [(Z) - 2 - (2 - amino - 4 - thiazolyl) - 2 - cyclopentyloxyiminoacétamide] - 4 - fluorométhyl - 2 - oxo - 1 - azétidinesulfonique.

12. Procédé suivant la revendication 1, dans lequel le composé répondant à la formule (1) est l'acide (3S, 4R) - (−) - 3 - [(Z) - 2 - (2 - amino - 4 - thiazolyl) - 2 - benzyloxyiminoacétamide] - 4 - fluorométhyl - 2 - oxo - 1 - azétidinesulfonique.

13. Procédé suivant la revendication 1, dans lequel le composé répondant à la formule (1) est l'acide (3S, 4R) - (−) - 3 - [(Z) - 2 - (2 - amino - 4 - thiazolyl) - 2 - phénoxyiminoacétamide] - 4 - fluorométhyl - 2 - oxo - 1 - azétidinesulfonique.

14. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - méthylpropoxyimino)acétamide] - 4 - fluorométhyl - 2 - oxo - 1 - azétidinesulfonique.

15. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - cyclopropoxyimino)acétamide] - 4 - fluorométhyl - 2 - oxo - 1 - azétidinesulfonique.

16. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - cyclobutoxyimino)acétamide] - 4 - fluorométhyl - 2 - oxo - 1 - azétidinesulfonique.

17. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - cyclopentoxyimino)acétamide] - 4 - fluorométhyl - 2 - oxo - 1 - azétidinesulfonique.

18. Procédé suivant la revendication 1, dans lequel le composé répondant à la formule (1) est l'acide (3S, 4R) - (−) - 3 - [(Z) - 2 - (2 - amino - 4 - thiazolyl) - 2 - (1 - carboxy - 1 - cyclohexyloxyimino)acétamide] - 4 - fluorométhyl - 2 - oxo - 1 - azétidinesulfonique.